# EUROPEAN PATENT APPLICATION

(11) **EP 4 624 047 A1**
(43) Date of publication of application: **01.10.2025**
(21) Application number: 25162901.0
(22) Date of filing: 11.03.2025
(51) Int. Cl.: B01L 3/00, B01L 7/00, B01L 9/00

(54) **CHAMBER DEVICE, HYBRIDIZATION REACTION SYSTEM, NUCLEIC ACID ANALYSIS SYSTEM, SEALING MEMBER, RESIN CHAMBER DEVICE, AND SAMPLE SETTING METHOD FOR RESIN CHAMBER DEVICE**

(30) Priority: 13.03.2024 JP 2024039115; 15.03.2024 JP 2024040909
(71) Applicant: Yokogawa Electric Corporation, Tokyo 180-8750 (JP)
(72) Inventor: Uchino, Youichi, Musashino-shi, Tokyo, 180-8750 (JP); Shiba, Mikio, Kawasaki-shi, Kanagawa, 214-0022 (JP); Kakuryu, Nobuyuki, Musashino-shi, Tokyo, 180-8750 (JP)
(74) Representative: Grünecker Patent- und Rechtsanwälte PartG mbB

(57) **Abstract**

A chamber device includes a chamber unit and a chamber frame. The chamber unit includes a sealing member in which a plurality of well forming portions penetrating through the sealing member in a thickness direction are formed; a transparent member that is attached to the sealing member on a side of a first surface from which ends of the well forming portions on one side are open, the transparent member blocking the ends of the well forming portions on the one side; and a substrate that is attached to the sealing member on a side of a second surface from which ends of the well forming portions on the other side are open, the substrate blocking the ends of the well forming portions on the other side. The chamber frame detachably pinches the chamber unit in an overlapping direction of the transparent member, the sealing member, and the substrate.

## Description

### BACKGROUND

### Technical Fields

The present invention relates to a chamber device, a hybridization reaction system, a nucleic acid analysis system, a sealing member, a resin chamber device, and a sample setting method for a resin chamber device. Priority is claimed on Japanese Patent Application No. 2024-039115, filed March 13, 2024 and Japanese Patent Application No. 2024-040909, filed March 15, 2024, the content of which is incorporated herein by reference.

### Description of Related Art

Japanese Translation of PCT International Application Publication No. 2009-542222 discloses a chamber device including: a chamber frame that includes an upper integrated gasket and a lower integrated gasket; a substrate; and a substrate frame positioning and capturing the substrate.

Japanese Unexamined Patent Application, First Publication No. 2009-082084 discloses a target substance detection device including, in a biochemical reaction cassette that includes a first flow path and a second flow path that establish communication between outside and a reaction chamber that includes a fixation region for a probe for detecting a target substance and is for causing a material to react in the fixation region; a tightly closing portion that is able to tightly close the first flow path; and a pressure alleviating section that keeps a pressure in the reaction chamber substantially constant.

There is room for improvement in assemblability and operability of the chamber device in the aforementioned related art. Also, since the aforementioned biochemical reaction cassette is obtained by bonding glass and a casing made of a resin, it is necessary to separate them to discard them, and it is difficult to dispose of them (throw them away).

A first object of the present invention is to improve assemblability and operability of a chamber device. A second object of the present invention is to make the chamber device disposable while securing air tightness of a chamber.

### SUMMARY

In order to solve the aforementioned problem, a chamber device according to a first aspect of the present invention includes: a chamber unit including a sealing member in which a plurality of well forming portions penetrating through the sealing member in a thickness direction are formed, a transparent member that is attached to the sealing member on a side of a first surface from which ends of the well forming portions on one side are open, the transparent member blocking the ends of the well forming portions on the one side, and a substrate that is attached to the sealing member on a side of a second surface from which ends of the well forming portions on the other side are open, the substrate blocking the ends of the well forming portions on the other side; and a chamber frame that detachably pinches the chamber unit in an overlapping direction of the transparent member, the sealing member, and the substrate.

In a chamber device according to a second aspect of the present invention, the chamber frame includes a first frame that abuts the chamber unit on the side of the first surface, a second frame that abuts the chamber unit on the side of the second surface, a hinge mechanism that turnably couples the first frame and the second frame, and a locking mechanism that locks both the first frame and the second frame in a state where both the frames lie on top of one another, in the chamber device according to the first aspect of the present invention.

In a chamber device according to a third aspect of the present invention, the locking mechanism includes a latch that is turnably provided in one of the first frame and the second frame and a caught portion that is provided in the other one of the first frame and the second frame and is caught by the latch, in the chamber device according to the second aspect of the present invention.

In a chamber device according to a fourth aspect of the present invention, plating treatment is performed on a surface of at least one of the latch and the caught portion, in the chamber device according to the third aspect of the present invention.

In a chamber device according to a fifth aspect of the present invention, annular projecting portions that abut the substrate and the transparent member in a compressed state when the chamber unit is pinched by the chamber frame are formed at peripheral edge portions of the openings of the well forming portions, in the chamber device according to any one of the first to fourth aspects of the present invention.

In a chamber device according to a sixth aspect of the present invention, first hole portions and second hole portions extending from inner wall surfaces of the well forming portions to outer surfaces are formed in the sealing member, in the chamber device according to any one of the first to fifth aspects of the present invention.

In a chamber device according to a seventh aspect of the present invention, steps or walls are formed between the first hole portions and the second hole portions on the inner wall surfaces of the well forming portions, in the chamber device according to the sixth aspect of the present invention.

In a chamber device according to an eighth aspect of the present invention, a lid member that is attached to the sealing member and blocks the first hole portions and the second hole portions is included, in the chamber device according to the sixth or seventh aspect of the present invention.

In a chamber device according to a ninth aspect of the present invention, the sealing member includes a first engagement groove that is formed on the side of the first surface and is engaged with a peripheral edge portion of the transparent member and a second engagement groove that is formed on the side of the second surface and is engaged with a peripheral edge portion of the substrate, in the chamber device according to any one of the first to eighth aspects of the present invention.

A hybridization reaction system according to a tenth aspect of the present invention performs a hybridization reaction using the chamber device according to any one of the first to ninth aspects of the present invention.

A nucleic acid analysis system according to an eleventh aspect of the present invention analyzes a nucleic acid extracted using the hybridization reaction system according to the tenth aspect of the present invention.

A sealing member according to a twelfth aspect of the present invention includes: a plurality of well forming portions that penetrate through the sealing member in a thickness direction; first hole portions and second hole portions that extend from inner wall surfaces of the well forming portions to outer surfaces; and steps or walls that are disposed between the first hole portions and the second hole portions on the inner wall surfaces of the well forming portions.

A resin chamber device according to a thirteenth aspect of the present invention includes: a sealing member in which well forming portions penetrating through the sealing member in a thickness direction are formed; a transparent member that is disposed on the sealing member on a side of a first surface from which ends of the well forming portions on one side are open, the transparent member blocking the ends of the well forming portions on the one side; a substrate that is disposed on the sealing member on a side of a second surface from which ends of the well forming portions on the other side are open, the substrate blocking the ends of the well forming portions on the other side; a first frame that abuts the transparent member; a second frame that abuts the substrate; and a clamp member that clamps peripheral edge portions of the first frame and the second frame.

In a resin chamber device according to a fourteenth aspect of the present invention, the first frame and the second frame are formed into rectangular shapes in a plan view, and a plurality of slide grooves that extend in a long-side direction from four corner portions and allow the clamp member to be engaged with the slide grooves are formed, in the resin chamber device according to the thirteenth aspect of the present invention.

In a resin chamber device according to a fifteenth aspect of the present invention, the slide grooves have a first inclined shape that becomes shallower toward an intermediate position of the first frame and the second frame in the long-side direction, and the clamp member has an engagement claw portion that is engaged with the slide grooves and has a second inclined shape corresponding to the first inclined shape, in the resin chamber device according to the fourteenth aspect of the present invention.

In a resin chamber device according to a sixteenth aspect of the present invention, a surface of the first frame is covered with jet black plating made of a resin, and a window portion to look into the well forming portions through the transparent member is formed in the first frame, in the resin chamber device according to any one of the thirteenth to fifteenth aspects of the present invention.

In a resin chamber device according to a seventeenth aspect of the present invention, one of the first frame and the second frame includes a positioning pin formed thereon to project toward the other, and a positioning hole with which the positioning pin is engaged is formed in the other one of the first frame and the second frame, in the resin chamber device according to any one of the thirteenth to fifteenth aspects of the present invention.

A hybridization reaction system according to an eighteenth aspect of the present invention performs a hybridization reaction using the resin chamber device according to any one of the thirteenth to fifteenth aspects of the present invention.

A nucleic acid analysis system according to a nineteenth aspect of the present invention analyzes a nucleic acid extracted using the hybridization reaction system according to the eighteenth aspect of the present invention.

A sample setting method for a resin chamber device according to a twentieth aspect of the present invention includes: a first process of forming a first chamber unit by causing a sealing member in which ends of well forming portions on one side are blocked with a transparent member to overlap a first frame, the well forming portions penetrating through the sealing member in a thickness direction; a second process of dropping a sample from ends of the well forming portions on the other side into the well forming portions after the first process; a third process of causing a second chamber unit formed by causing a substrate to overlap a second frame to overlap the first chamber unit and blocking the ends of the well forming portions on the other side with the substrate after the second process; and a fourth process of clamping peripheral edge portions of the first frame and the second frame with a clamp member after the third process.

According to an aspect of the present invention, it is possible to improve assemblability and operability of the chamber device. Also, according to an aspect of the present invention, it is possible to make the chamber device disposable while securing air tightness of the chamber.

Further features and aspects of the present invention will become obvious from detailed description of embodiments given below with reference to the accompanying drawings.

### BRIEF DESCRIPTION OF THE DRAWINGS

FIG. 1 is a perspective view of a chamber device according to a first embodiment of the present invention.
FIG. 2 is a right side view of the chamber device according to the first embodiment of the present invention.
FIG. 3 is a perspective view illustrating a state where a chamber frame has been opened and a cap has been removed according to the first embodiment of the present invention.
FIG. 4 is a perspective view illustrating how a chamber unit is attached to the chamber frame according to the first embodiment of the present invention.
FIG. 5 is an exploded perspective view of the chamber unit according to the first embodiment of the present invention.
FIG. 6 is a plan view of the chamber unit according to the first embodiment of the present invention.
FIG. 7 is a bottom view of the chamber unit according to the first embodiment of the present invention.
FIG. 8 is a sectional view seen along the arrow VIII-VIII illustrated in FIG. 6.
FIG. 9 is a flow diagram of assembly of the chamber device according to the first embodiment of the present invention.
FIG. 10 is a schematic view of a nucleic acid analysis system according to the first embodiment of the present invention.
FIG. 11 is a perspective view of a well forming portion according to a second embodiment of the present invention.
FIG. 12 is a plan view of a chamber device according to a third embodiment of the present invention.
FIG. 13 is a bottom view of the chamber device according to the third embodiment of the present invention.
FIG. 14 is a perspective view of a chamber device according to a fourth embodiment of the present invention.
FIG. 15 is an exploded perspective view of the chamber device according to the fourth embodiment of the present invention.
FIG. 16 is a sectional view of the chamber device according to the fourth embodiment of the present invention.
FIG. 17 is an enlarged view of a region A illustrated in FIG. 16.
FIG. 18 is a perspective view of a resin chamber device according to a fifth embodiment of the present invention.
FIG. 19 is a front view of the resin chamber device according to the fifth embodiment of the present invention.
FIG. 20 is an exploded perspective view of the resin chamber device according to the fifth embodiment of the present invention.
FIG. 21 is a sectional view of the resin chamber device according to the fifth embodiment of the present invention.
FIG. 22 is a flow diagram of a sample setting method of the resin chamber device according to the fifth embodiment of the present invention.
FIG. 23 is an assembly diagram of the resin chamber device in accordance with the flow diagram illustrated in FIG. 22.
FIG. 24 is an assembly diagram of the resin chamber device in accordance with the flow diagram illustrated in FIG. 22.
FIG. 25 is an assembly diagram of the resin chamber device in accordance with the flow diagram illustrated in FIG. 22.
FIG. 26 is an assembly diagram of the resin chamber device in accordance with the flow diagram illustrated in FIG. 22.
FIG. 27 is a schematic view of a nucleic acid analysis system using the resin chamber device according to the fifth embodiment of the present invention.
FIG. 28 is a side view of a resin chamber device according to a sixth embodiment of the present invention.
FIG. 29 is an exploded perspective view of a resin chamber device according to a seventh embodiment of the present invention.
FIG. 30 is a sectional view of the resin chamber device according to the seventh embodiment of the present invention.

### DETAILED DESCRIPTION OF THE EMBODIMENTS

Hereinafter, a chamber device, a hybridization reaction system, a nucleic acid analysis system, and a sealing member according to first to fourth embodiments of the present invention will be described in detail with reference to the drawings. Hereinafter, an overview of the embodiments of the present invention will be described first, and details of each embodiment of the present invention will then be described.

### [Overview]

Japanese Translation of PCT International Application Publication No. 2009-542222 discloses a method of fastening and sealing upper and lower hybridization chamber parts in a whole circumference snapping scheme. Also, Japanese Unexamined Patent Application, First Publication No. 2009-082084 discloses a hybridization chamber technology of providing flow paths to inject a test piece at two locations, stirring the test piece to improve reaction efficiency, and not inhibiting a flow of the test piece by an effect of a pressure alleviating section that keeps a pressure in a reaction chamber substantially constant.

However, in the configuration described in Japanese Translation of PCT International Application Publication No. 2009-542222, the whole circumference is fastened with a snap after pouring the test piece into each cell, the injected test piece may spill, and it may be necessary to perform assembly so as not to cause contamination with other cells. Also, in a case where it is necessary to perform dismantling after assembling, it may be difficult to dismantle the hybridization chamber parts due to the whole circumference snapping fastening. Furthermore, the sealed test piece or the like may scatter and cleaning may be needed when the hybridization chamber parts are dismantled. Also, since glass and the casing are bonded in the configuration described in Japanese Unexamined Patent Application, First Publication No. 2009-082084, manufacturing costs may increase.

In the embodiments of the present invention, a chamber unit is assembled by causing a transparent member, a sealing member, and a substrate to overlap one another, and the chamber unit is detachably pinched by a chamber frame. With this configuration, it is possible to configure a chamber device by parts that are not bonded, and assemblability and operability of the chamber device are improved. Also, it is possible to repeatedly use the chamber frame without cleaning it by detaching and exchanging the chamber unit after use. Furthermore, it is possible to prevent the sealed test piece from scattering by discarding the chamber unit after the exchanging as it is.

### [First embodiment]

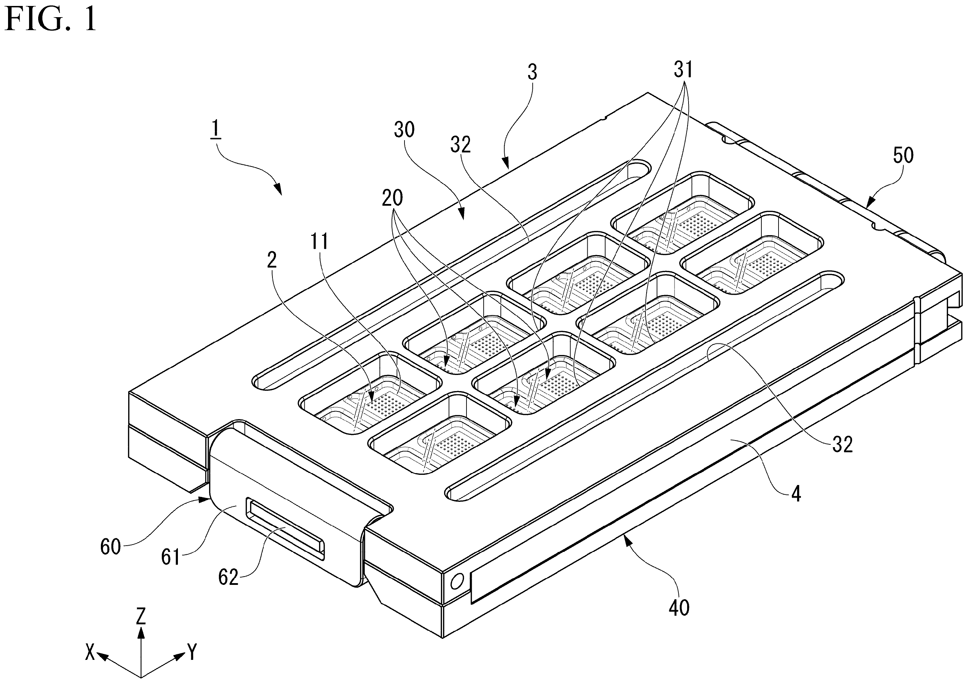

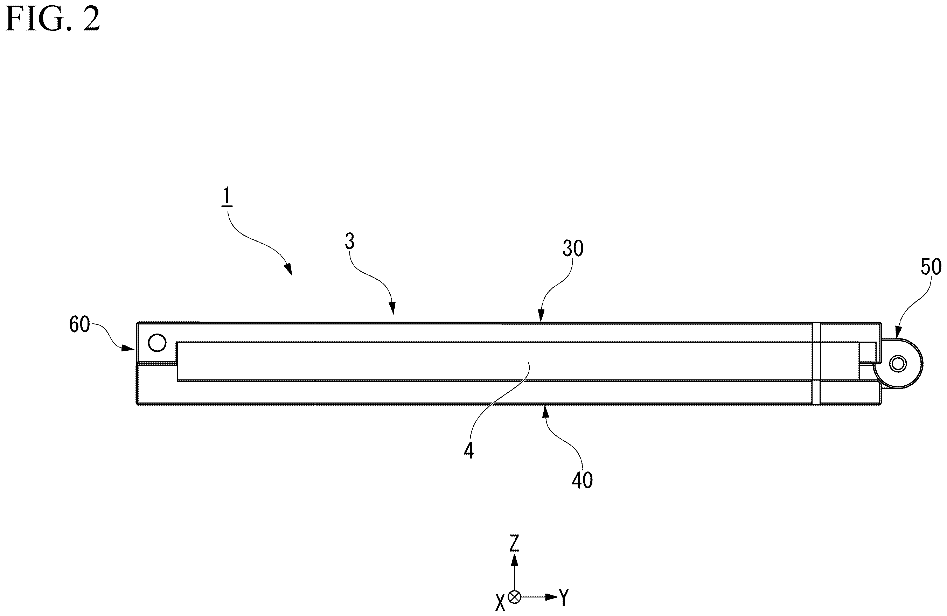

FIG. 1 is a perspective view of a chamber device 1 according to the first embodiment of the present invention. FIG. 2 is a right side view of the chamber device 1 according to the first embodiment of the present invention.

As illustrated in the drawings, the chamber device 1 includes a chamber unit 2, a chamber frame 3, and caps 4 (lid members). The chamber device 1 has a flat and substantially rectangular parallelepiped shape.

Note that an XYZ orthogonal coordinate system may be set and positional relationships of members may be described with reference to the XYZ orthogonal coordinate system in the following description. An X-axis direction is a width direction (left-right direction) of the chamber device 1. A Y-axis direction is a depth direction (front-rear direction) of the chamber device 1. A Z-axis direction is a thickness direction (up-down direction) of the chamber device 1. Note that on the assumption that the side of the chamber frame 3 on which a locking mechanism 60 is disposed is a front side, and the side on which a hinge mechanism 50 is disposed is a rear side, the front and rear sides, the upper and lower sides, and the left and right sides of the chamber device 1 will be defined.

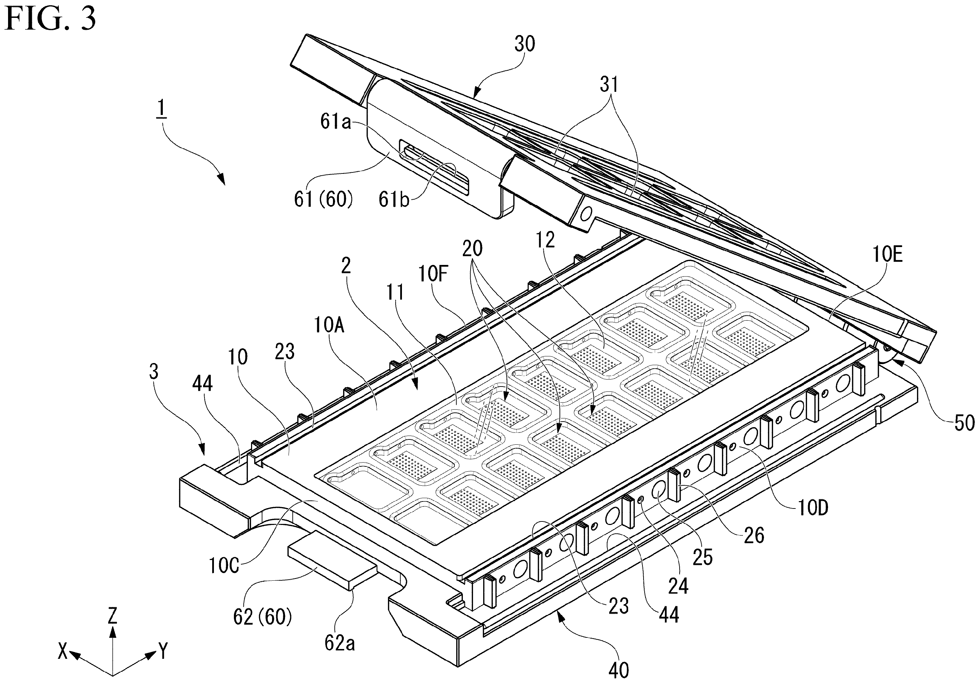

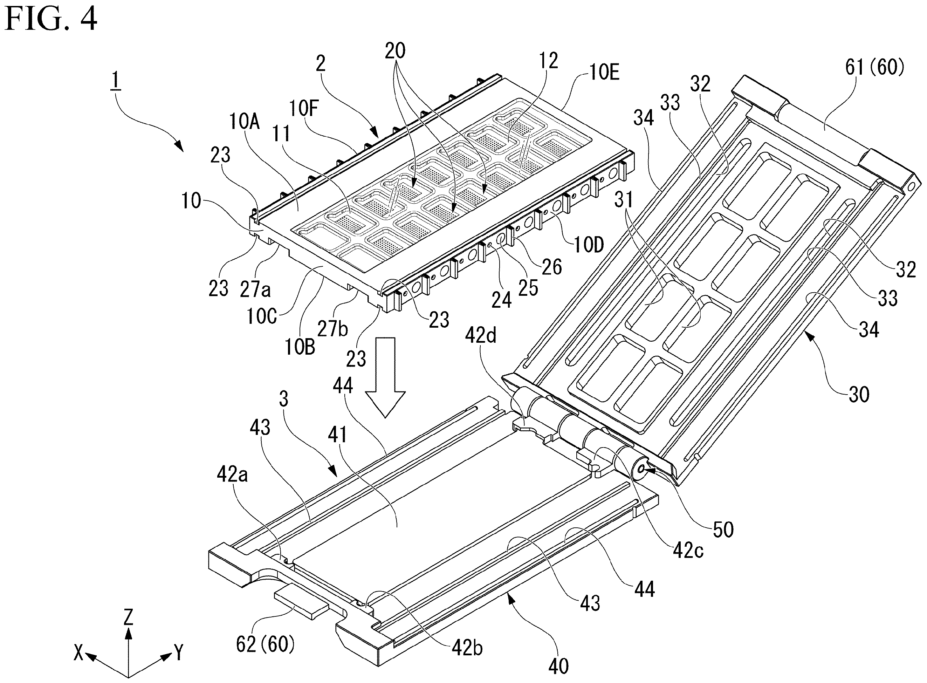

FIG. 3 is a perspective view illustrating a state where the chamber frame 3 has been opened and the caps 4 have been removed according to the first embodiment of the present invention. FIG. 4 is a perspective view illustrating how the chamber unit 2 is attached to the chamber frame 3 according to the first embodiment of the present invention.

As illustrated in these drawings, the chamber unit 2 is detachably pinched by the chamber frame 3 in the chamber device 1. A plurality of well forming portions 20 that accommodate test pieces are formed in the chamber unit 2.

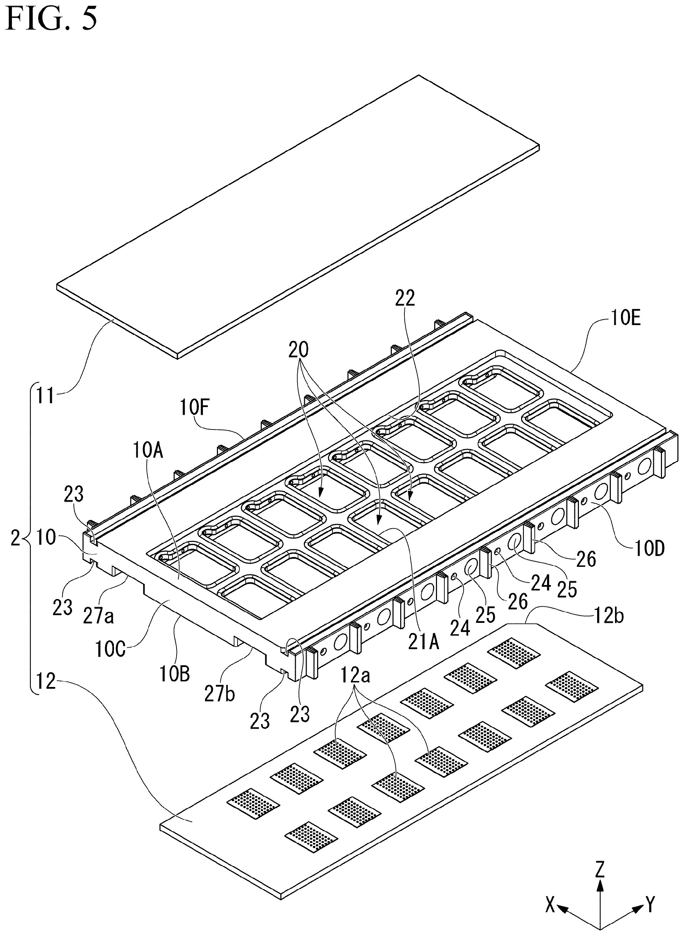

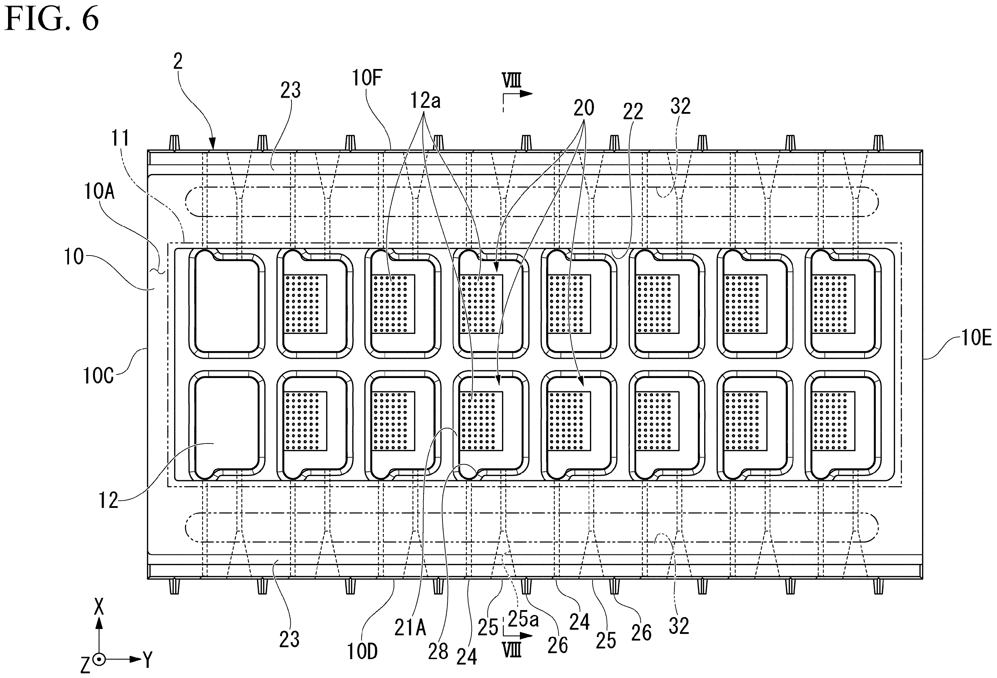

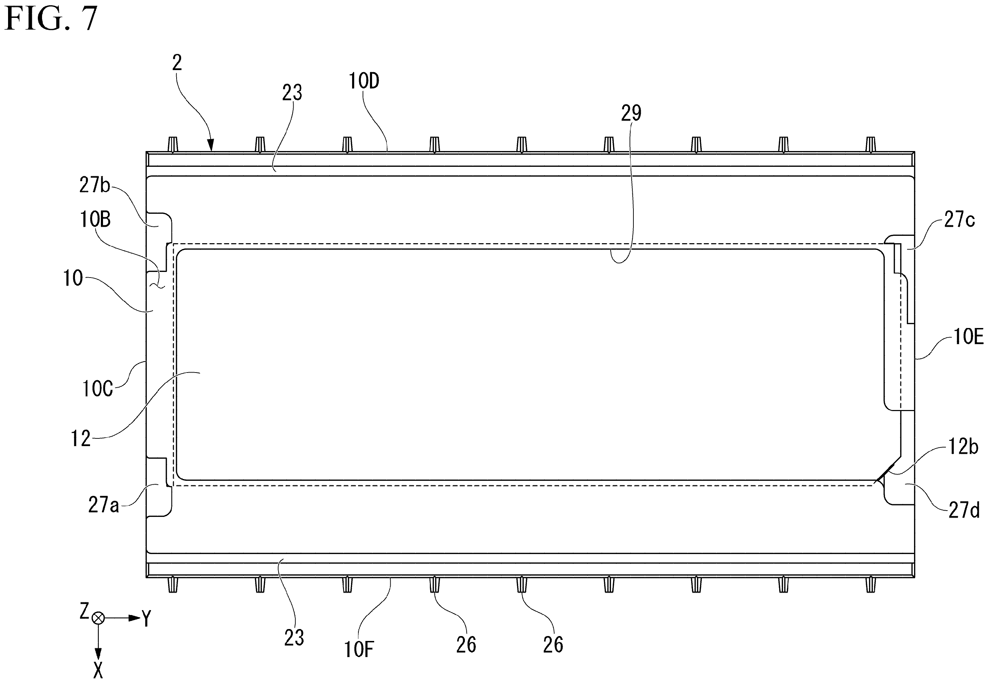

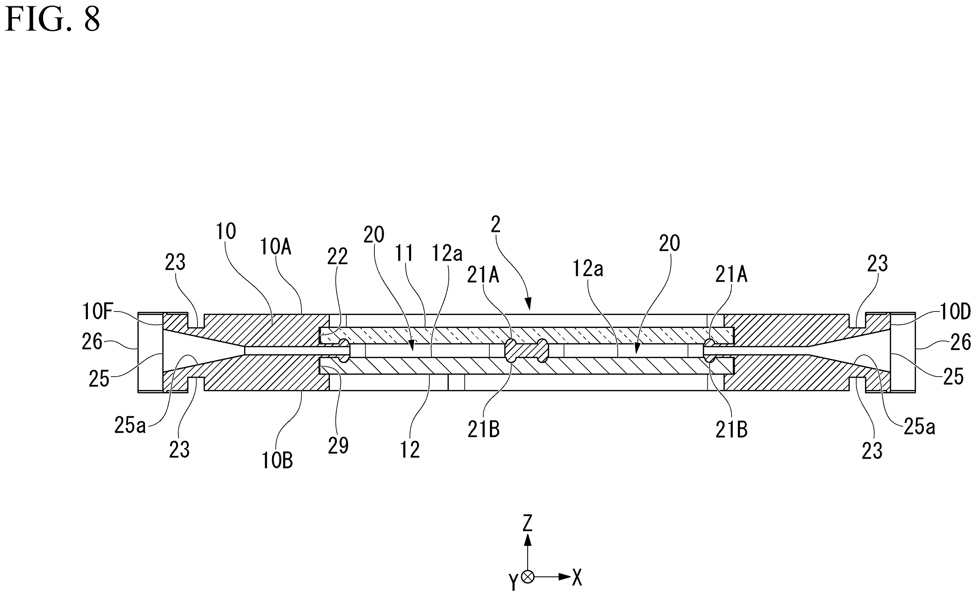

FIG. 5 is an exploded perspective view of the chamber unit 2 according to the first embodiment of the present invention. FIG, 6 is a plan view of the chamber unit 2 according to the first embodiment of the present invention. FIG. 7 is a bottom view of the chamber unit 2 according to the first embodiment of the present invention. FIG. 8 is a sectional view seen along the arrow VIII-VIII illustrated in FIG. 6.

As illustrated in FIG. 5, the chamber unit 2 includes a sealing member 10, a slide glass 11 (transparent member), and a DNA array substrate 12 (substrate).

The sealing member 10 has a flat shape that is rectangular in a plan view. The sealing member 10 is formed of silicone rubber with elasticity, for example. The sealing member 10 includes a first surface 10A facing upward, a second surface 10B facing downward, a third surface 10C facing forward, a fourth surface 10D facing rightward, a fifth surface 10E facing backward, and a sixth surface 10F facing leftward.

A plurality of well forming portions 20 penetrating through the sealing member 10 in the thickness direction (Z-axis direction) are formed in the sealing member 10. The well forming portions 20 form columns in the depth direction (Y-axis direction) and form two columns at an interval therebetween in the width direction (X-axis direction). Note that the number, arrangement, and the shape of the well forming portions 20 are not limited to those illustrated in the drawing.

The slide glass 11 is attached to the sealing member 10 on the side of the first surface 10A from which ends (upper ends) of the well forming portions 20 on one side are open. The slide glass 11 blocks the openings at the ends (upper ends) of the well forming portions 20 on the one side in a state where the slide glass 11 is attached to the sealing member 10. As the slide glass 11, it is possible to use a commercially available standardized product. Note that this component is not limited to the slide glass 11 as long as the component is a transparent member with a rectangular shape in a plan view, and a transparent acrylic plate or the like may be used.

The DNA array substrate 12 includes a plurality of DNA microarrays 12a in which probe carriers for causing a hybridization reaction are arranged in an array shape. The DNA microarrays 12a are formed in an arrangement corresponding to the well forming portions 20. Note that the number of the DNA microarrays 12a to be formed is not necessarily the same as the number of well forming portions 20. Also, in a case where a reaction other than the hybridization reaction is performed, a substrate including reaction portions corresponding thereto may be used.

The DNA array substrate 12 is attached to the sealing member 10 on the side of the second surface 10B from which the ends (lower ends) of the well forming portions 20 on the other side are open. The DNA array substrate 12 blocks the openings of the ends (lower ends) of the well forming portions 20 on the other side in a state where the DNA array substrate 12 is attached to the sealing member 10. A notch portion 12b for preventing an assembling (orientation) error with respect to the sealing member 10 is formed in the DNA array substrate 12. The notch portion 12b is formed at one of four corner portions of the DNA array substrate 12.

As illustrated in FIG. 6, annular projecting portions 21A to abut the slide glass 11 are formed at peripheral edge portions of the openings of the well forming portions 20 on the side of the first surface 10A of the sealing member 10. The annular projecting portions 21A have a projecting shape that is semicircular in a sectional view as illustrated in FIG. 8. The annular projecting portions 21A abut the slide glass 11 attached to the sealing member 10 in a compressed state in the up-down direction (Z-axis direction) to thereby enhance air rightness of the well forming portions 20.

Also, a first engagement groove 22 that is engaged with a peripheral edge portion of the slide glass 11 is formed in the sealing member 10 on the side of the first surface 10A. The first engagement groove 22 is engaged over the whole circumference of the peripheral edge portion of the slide glass 11 as illustrated in FIG. 6. Note that when the slide glass 11 is engaged with the first engagement groove 22, the sealing member 10 is elastically deformed.

As illustrated in FIG. 5, engagement recessed portions 23 extending in the front-rear direction (Y-axis direction) are formed in the first surface 10A of the sealing member 10. The engagement recessed portions 23 are formed in a pair along the fourth surface 10D and the sixth surface 10F. Moreover, engagement recessed portions 23 extending in the front-rear direction (Y-axis direction) are also formed in the second surface 10B of the sealing member 10. The engagement recessed portions 23 are also formed in a pair along the fourth surface 10D and the sixth surface 10F.

As illustrated in FIG. 6, injection ports 25 (first hole portions) and discharge ports 24 (second hole portions) extending in the width direction (X-axis direction) from the inner wall surfaces of the well forming portions 20 to the outer surfaces (the fourth surface 10D and the sixth surface 10F) of the sealing member 10 are formed in the sealing member 10. Injection needles such as pipettors for injecting test pieces into the well forming portions 20 are inserted into the injection ports 25. Air, test pieces, and the like are discharged from the well forming portions 20 through the discharge ports 24.

Steps 28 are formed between the injection ports 25 and the discharge ports 24 on the inner wall surfaces of the well forming portions 20. In other words, dents are provided in the inner wall surfaces of the well forming portions 20, and the discharge ports 24 are formed in the dents. It is thus possible to reduce a reagent injected from the injection ports 25 into the well forming portion 20 being short-circuited with the discharge ports 24 immediately after the pouring and being discharged therefrom. Therefore, it is possible to efficiently pour a specific amount of reagent into the well forming portions 20.

The injection ports 25 on the outlet side (outer surface side) are tapered portions 25a with increased diameters. Plate portions 26 laterally projecting are formed on the outer surfaces (the fourth surface 10D and the sixth surface 10F) of the sealing member 10. The plate portions 26 are provided to separate an injection port 25 and a discharge port 24 communicating with the same well forming portion 20 from an injection port 25 and a discharge port 24 communicating with another well forming portion 20 to prevent contamination of test pieces between the well forming portions 20.

The caps 4 illustrated in FIGS. 1 and 2 are attached to the outer surfaces (the fourth surface 10D and the sixth surface 10F) of the sealing member 10 and block the injection ports 25 and the discharge ports 24. The caps 4 include two types of projecting portions (not illustrated) that can be inserted into the injection ports 25 and the discharge ports 24 and accommodating grooves (not illustrated) to accommodate the plate portions 26.

As illustrated in FIG. 8, annular projecting portions 21B abutting the DNA array substrate 12 are formed at peripheral edge portions of the openings of the well forming portions 20 on the side of the second surface 10B of the sealing member 10. The annular projecting portions 21B have a projecting shape that is semicircular in a sectional view. The annular projecting portions 21B abut the DNA array substrate 12 attached to the sealing member 10 in a compressed state in the up-down direction (Z-axis direction) to thereby enhance air tightness of the well forming portions 20.

A second engagement groove 29 that is engaged with the peripheral edge portion of the DNA array substrate 12 is formed on the side of the second surface 10B of the sealing member 10. The second engagement groove 29 is engaged with substantially the whole circumference of the peripheral edge portion of the DNA array substrate 12 as illustrated in FIG. 7. Note that when the DNA array substrate 12 is engaged with the second engagement groove 29, the sealing member 10 is elastically deformed.

Positioning recessed portions 27a to 27d are formed on the side of the second surface 10B of the sealing member 10 at positions corresponding to four corner portions of the DNA array substrate 12. The positioning recessed portions 27a and 27b are formed at positions corresponding to the two front corner portions of the DNA array substrate 12. The positioning recessed portions 27c and 27d are formed at positions corresponding to the two rear corner portions of the DNA array substrate 12.

The positioning recessed portions 27c and 27d communicate with the second engagement groove 29. Therefore, the two rear corner portions of the DNA array substrate 12 extend from the inside of the second engagement groove 29 to the inside of the positioning recessed portions 27c and 27d. Note that although the positioning recessed portions 27a and 27b do not communicate with the second engagement groove 29, the positioning recessed portions 27a and 27b may communicate therewith.

As illustrated in FIG. 4, the chamber frame 3 includes a first frame 30 that abuts the chamber unit 2 on the side of the first surface 10A, a second frame 40 that abuts the chamber unit 2 on the side of the second surface 10B, a hinge mechanism 50 that turnably couples the first frame 30 and the second frame 40, and a locking mechanism 60 that locks the first frame 30 and the second frame 40 in a state where both the frames lie on top of one another.

A support base 41 that supports the DNA array substrate 12 from the lower side and positioning projecting portions 42a to 42d provided at four locations around the support base 41 are formed in the second frame 40. The support base 41 has a rectangular shape in a plan view and has a size with which the support base 41 can be inserted into the second engagement groove 29 illustrated in FIG. 7.

The positioning projecting portions 42a to 42d have a size with which the positioning projecting portions 42a to 42d can be inserted into the positioning recessed portions 27a to 27d illustrated in FIG. 7. The DNA array substrate 12 (chamber unit 2) is positioned with respect to the second frame 40 by the positioning projecting portions 42a to 42d being inserted into the positioning recessed portions 27a to 27d. Note that a dent to avoid interference with each corner portion of the DNA array substrate 12 is formed in each of the positioning projecting portions 42a to 42d as illustrated in FIG. 4.

A pair of engagement ribs 43 extending in parallel to the front-rear direction (Y-axis direction) are formed on both the left and right sides of the support base 41. The pair of engagement ribs 43 are engaged with a pair of engagement recessed portions 23 provided in the aforementioned sealing member 10 on the side of the second surface 10B. The sealing member 10 (chamber unit 2) is positioned with respect to the second frame 40 by the pair of engagement ribs 43 being engaged with the pair of engagement recessed portions 23.

A pair of pinching projecting portions 44 extending in parallel to the front-rear direction (Y-axis direction) are formed further on both the left and right sides of the pair of engagement ribs 43. The pair of pinching projecting portions 44 are projecting portions that pinch the caps 4 illustrated in FIGS. 1 and 2. In this manner, the caps 4 are not easily separated from the sealing member 10 (chamber unit 2).

As illustrated in FIG. 4, a plurality of window portions 31 for observing the well forming portions 20 and a pair of slits 32 disposed on both the left and right sides of the plurality of window portions 31 are formed in the first frame 30. The plurality of window portions 31 are formed at positions where the window portions 31 overlap the well forming portions 20 in the Z-axis direction in a state where the chamber device 1 is assembled as illustrated in FIG. 1.

The window portions 31 penetrate through the first frame 30 in the thickness direction (Z-axis direction). The window portions 31 form columns in the depth direction (Y-axis direction) and are formed in two columns at an interval in the width direction (X-axis direction). Note that the number, the arrangement, and the shape of the window portions 31 are not limited to those illustrated in the drawing. In the present embodiment, two well forming portions 20 can be observed from one window portion 31.

The slits 32 penetrate through the first frame 30 in the thickness direction (Z-axis direction). The slits 32 are formed at positions where the slits 32 overlap the intermediate portions of the injection ports 25 and the discharge ports 24 in the sealing member 10 in a plan view as illustrated in FIG. 6. In this manner, it is possible to confirm that the injection needles are reliably inserted into the injection ports 25 when test pieces are injected into the well forming portions 20. Note that the sealing member 10 may have light translucency and may be transparent or semitransparent, for example.

As illustrated in FIG. 4, a pair of engagement ribs 33 extending in parallel to the front-rear direction (Y-axis direction) are formed further on both the left and right sides of the pair of slits 32. The pair of engagement ribs 33 are engaged with the pair of engagement recessed portions 23 provided in the aforementioned sealing member 10 on the side of the first surface 10A. The sealing member 10 (chamber unit 2) is positioned with respect to the first frame 30 by the pair of engagement ribs 33 being engaged with the pair of engagement recessed portions 23.

A pair of pinching projecting portions 34 extending in parallel to the front-rear direction (Y-axis direction) are formed further on both the left and right sides of the pair of engagement ribs 33. The pair of pinching projecting portions 34 are projecting portions that pinch the caps 4 illustrated in FIGS. 1 and 2. In this manner, the caps 4 are not easily separated from the sealing member 10 (chamber unit 2).

The hinge mechanism 50 couples end portions of the first frame 30 and the second frame 40 on the rear side such that the first frame 30 and the second frame 40 are able to turn around an axis extending in the width direction (X-axis direction) as illustrated in FIG. 1.

The locking mechanism 60 fixes end portions of the first frame 30 and the second frame 40 on the front side such that locking can be released as illustrated in FIGS. 3 and 4.

The locking mechanism 60 includes a latch 61 provided in the first frame 30 such that the latch 61 is able to turn and a caught portion 62 that is provided in the second frame 40 and is caught by the caught portion 62. Note that the latch 61 may be provided in the second frame 40 such that the latch 61 is able to turn while the caught portion 62 may be provided in the first frame 30.

The latch 61 is coupled to the front end portion of the first frame 30 such that the latch 61 is able to turn around an axis extending in the width direction (X-axis direction) as illustrated in FIG. 3. A long hole 61a extending in the width direction is formed in the latch 61. A claw portion 61b is provided to project from the inside of the long hole 61a. The claw portion 61b is formed on an inner wall surface of the long hole 61a on the side of the turning end of the latch 61 (lower side) and extends in the width direction.

The caught portion 62 is formed at a front end portion of the second frame 40. The caught portion 62 is formed into a plate shape extending in the width direction. A step 62a is formed to project on the side of the lower surface of the caught portion 62. The first frame 30 and the second frame 40 are locked by the caught portion 62 being inserted into the long hole 61a of the latch 61, the claw portion 61b crossing the step 62a, and the claw portion 61b being caught by the step 62a in the depth direction (Y-axis direction).

A fixing force caused by the locking mechanism 60 is preferably 20 N to 40 N, which is a range of a typical pinching force of human's fingers. The fixing force in the present embodiment is designed to be 20 N, for example. Also, surfaces of the latch 61 and the caught portion 62 are preferably subjected to plating treatment in order to improve slidability and durability. This enables smooth opening and closing operations of the chamber frame 3.

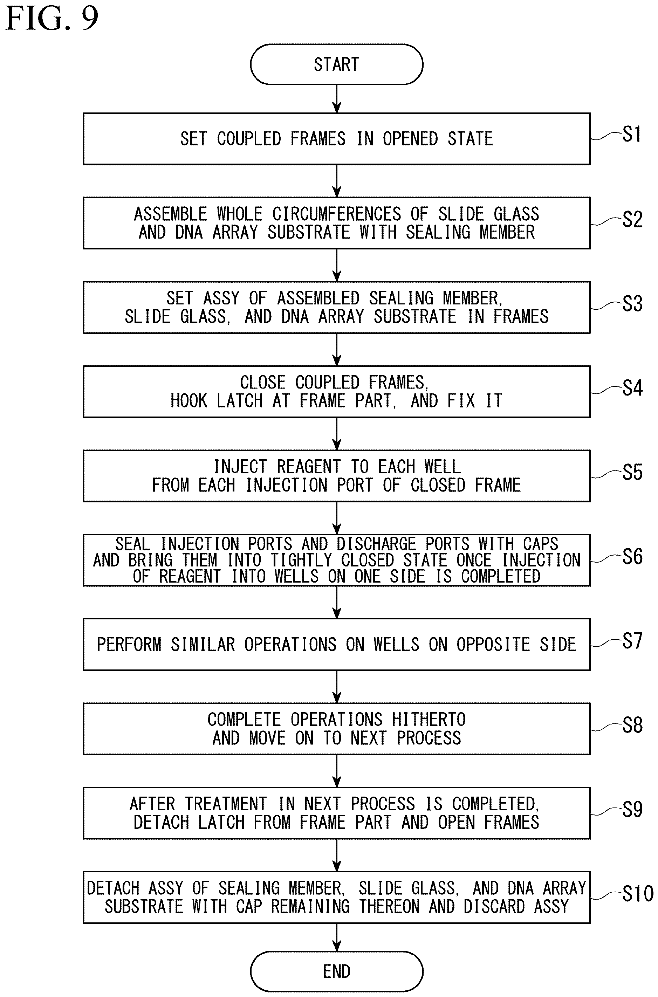

FIG. 9 is a flow diagram of assembly of the chamber device 1 according to the first embodiment of the present invention.

In a case where the aforementioned chamber device 1 is assembled, the first frame 30 and the second frame 40 coupled with the hinge mechanism 50 are set (stationarily placed) in an opened state first (Step S1).

Next, the whole circumferences of the slide glass 11 and the DNA array substrate 12 are assembled with the first engagement groove 22 and the second engagement groove 29 of the sealing member 10 (Step S2). The annular projecting portions 21A and 21B are formed at the peripheral edge portions of the openings of the well forming portions 20 in the first surface 10A and the second surface 10B of the sealing member 10, and it is possible to form tightly closed spaces of the well forming portions 20, that is, reaction spaces between the DNA microarray 12a and a reagent through the assembly of the slide glass 11 and the DNA array substrate 12.

Next, the chamber unit 2 with the sealing member 10, the slide glass 11, and the DNA array substrate 12 assembled therein is set in the second frame 40 (Step S3). The second frame 40 is provided with positioning structures (such as the positioning projecting portions 42a to 42d and the engagement ribs 33) to set the chamber unit 2 at a predetermined position such that the chamber unit 2 can be simply set.

Next, the first frame 30 coupled to the second frame 40 is closed, and the latch 61 is hooked at and fixed to the caught portion 62 in a state where the first frame 30 and the second frame 40 lie on top of one another (Step S4). The chamber frame 3 is held in a hand or is set on a dedicated jig or the like, and the reagent for inspection is injected from the injection ports 25 into the well forming portions 20 using a pipettor or the like (Step S5).

Once the injection of the reagent into the well forming portions 20 on one side (for example, the right side) is completed, the injection ports 25 and the discharge ports 24 on the one side are sealed with the cap 4, and the well forming portions 20 on the one side are brought into a tightly closed state (Step S6). Similar operations are performed on the well forming portions 20 on the opposite side (for example, the left side) as well (Step S7). Once all the above processes are completed, the processing proceeds to the next process (Step S8).

After the processing in the next process is completed, the latch 61 is detached from the caught portion 62, and the chamber frame 3 is opened (Step S9). Then, the chamber unit 2 with the sealing member 10, the slide glass 11, and the DNA array substrate 12 assembled therein is detached from the chamber frame 3 with the caps 4 remaining thereon and is then discarded as it is (Step S10). Since the whole circumferences or substantially the whole circumferences of the slide glass 11 and the DNA array substrate 12 are assembled with the sealing member 10, and the reagent therein can be held without any leakage for several tens of seconds, the reagent does not scatter to the chamber frame 3. It is thus possible to repeatedly use the chamber frame 3 without cleaning it.

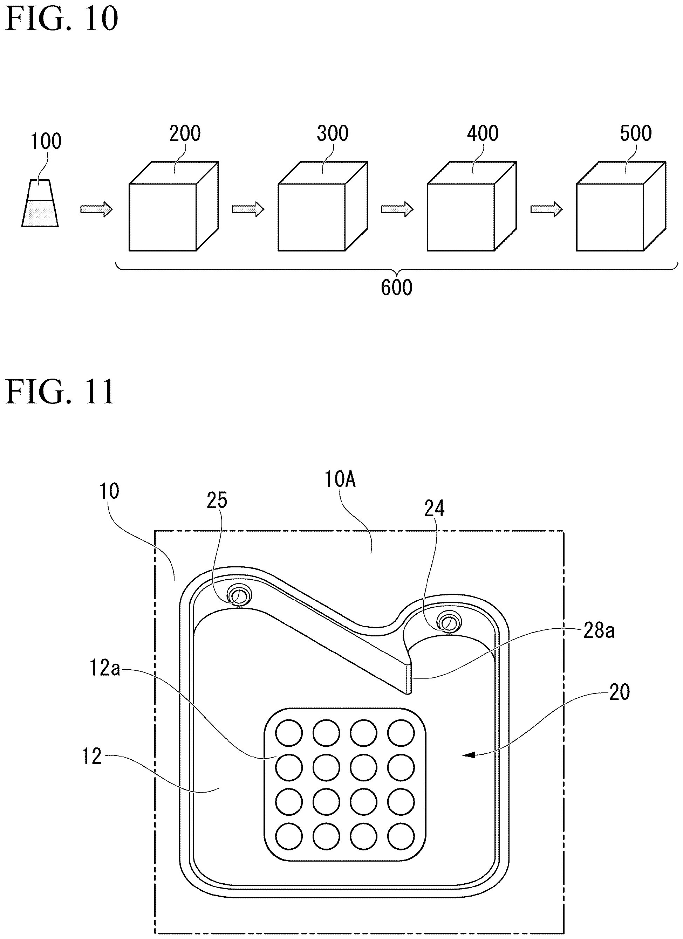

FIG. 10 is a schematic view of a nucleic acid analysis system 600 using the chamber device 1 according to the first embodiment of the present invention.

As illustrated in FIG. 10, the nucleic acid analysis system 600 includes a germ recovery system 200, a nucleic acid extraction system 300, a hybridization reaction system 400, and a detection system 500.

The germ recovery system 200 is a system that collects bacteria (such as bacteria and fungi) contained in a sample 100 from the sample 100. The sample 100 is, for example, a manufactured beverage, water to manufacture the beverage, or a solution in a process of manufacturing the beverage in a case of an inspection targeted at a beverage. Alternatively, the sample 100 may be a solution obtained by collecting germs from a cotton swab wiping off an inspection environment in order to inspect whether or not a manufacturing environment is contaminated by germs or a degree of contamination.

Germs can be collected through filtration using a filter or the like by pressurizing or depressurizing the collected solution, for example. In a case where bacteria or fungi are collected, for example, a pore diameter of the filter may be 0.22 µm to 0.45 µm. After the germs are collected by the filter, the filter is accommodated in a culture container, which will be described later, and is dipped into a culture solution in which the germs are cultivated to thereby cultivate the germs. The cultivation of the germs is, for example, static culture in which the culture container is stationarily placed to cultivate the germs or shaking culture in which the cultivation is performed while the culture container is shaken. The culture solution with the germs cultivated therein is transferred to the next process (nucleic acid extraction system 300). Note that the germs may be collected through centrifugation and be transferred to the next process, or the solution with the filter therein may be shaken and the solution with germs suspended therein may be transferred to the next process.

The nucleic acid extraction system 300 is a system that breaks (dissolves) membrane structures in cells in the solution and extracts nucleic acids of the cells of the germs. Note that a solution containing a different nucleic acid that reacts with the extracted nucleic acid may be mixed into the sample 100 from which the nucleic acid has been extracted. Also, the different nucleic acid may be a nucleic acid to which a site that exhibits a fluorescent, light-emitting, or light extinction effect under a specific condition is applied in order to be detected in a detection process (detection system 500), which will be described later. These may be mixed into the sample 100 before the nucleic acid extraction system 300 performs the treatment, or may be mixed into the sample 100 after the nucleic acid extraction system 300 performs the treatment.

The hybridization reaction system 400 is a system that causes the nucleic acid in the sample 100 to cause a hybridization reaction. In the process, the aforementioned chamber device 1 is used. In the hybridization reaction, the sample 100 causes a hybridization reaction that matches the aforementioned different nucleic acid by being heated to 60°C, for example, and stirred in the well forming portions 20. Through the reaction, the site that exhibits the fluorescent, light emitting, or light extinction effect under the specific condition applied to the aforementioned different nucleic acid reacts the nucleic acid in the sample 100, for example, and the fluorescent, light-emitting, or light extinction effect is thereby expressed.

Note that it is possible to cause the aforementioned different nucleic acid to react only with a nucleic acid that a specific germ in the sample 100 has, for example, by designing the structure of the aforementioned different nucleic acid to react with the specific nucleic acid. In other words, it is possible to express the fluorescent, light-emitting, or light extinction effect applied to the different nucleic acid only when the sample 100 contains the specific germ by using the different nucleic acid that reacts with the specific nucleic acid in the treatment performed by the hybridization reaction system 400.

The detection system 500 detects whether or not the fluorescent, light-emitting, or light extinction effect has been expressed in the sample 100 treated by the hybridization reaction system 400, a degree thereof, and the like. The detection system 500 excites the fluorescent effect expressed by the nucleic acid in the sample 100 with excitation laser light, for example, and the fluorescent light after the excitation is detected by a high-sensitivity camera.

Alternatively, the detection system 500 detects the light-emitting effect expressed by the nucleic acid in the sample 100 by a high-sensitivity camera. Alternatively, the detection system 500 detects a degree of light extinction of the fluorescent light or emitted light applied to the vicinity of the site to which the light extinction effect is applied by a high-sensitivity camera. In regard to the detection method, a method as described in Japanese Unexamined Patent Application, First Publication No. 2020-74726, for example, may be employed.

The nucleic acid analysis system 600 can analyze whether a specific germ (such as bacteria or fungi) is contained in the sample 100 or the concentration thereof by using the series of systems as described above.

As described above, the chamber device 1 according to the present embodiment includes: the chamber unit 2 that includes the sealing member 10 in which the plurality of well forming portions 20 penetrating through the sealing member 10 in the thickness direction are formed, the slide glass 11 (transparent member) that is attached to the sealing member 10 on the side of the first surface 10A from which the ends of the well forming portions 20 on one side are opened, the slide glass 11 blocking the ends of the well forming portions 20 on the one side; and the DNA array substrate 12 (substrate) that is attached to the sealing member 10 on the side of the second surface 10B from which the ends of the well forming portions 20 on the other side are open, the DNA array substrate 12 blocking the ends of the well forming portions 20 on the other side; and the chamber frame 3 that detachably pinches the chamber unit 2 in an overlapping direction of the slide glass 11, the sealing member 10, and the DNA array substrate 12. With this configuration, it is possible to configure the chamber device 1 by parts that are not bonded, and assemblability and operability of the chamber device 1 are improved. Also, it is possible to repeatedly use the chamber frame 3 without cleaning it by detaching and exchanging the chamber unit 2 after use. Furthermore, it is possible to prevent the sealed test pieces from scattering by discarding the exchanged chamber unit 2 as it is.

Also, in the chamber device 1 according to the present embodiment, the chamber frame 3 includes the first frame 30 that abuts the chamber unit 2 on the side of the first surface 10A, the second frame 40 that abuts the chamber unit 2 on the side of the second surface 10B, the hinge mechanism 50 that turnably couples the first frame 30 and the second frame 40, and the locking mechanism 60 that locks both the first frame 30 and the second frame 40 in a state where both the frames lie on top of one another. With this configuration, the first frame 30 and the second frame 40 are coupled with the hinge mechanism 50, it is necessary to fix both the frames at one location, which makes the assembling operation of the chamber device 1 simple.

Also, in the chamber device 1 according to the present embodiment, the locking mechanism 60 includes the latch 61 that is turnably provided in one of the first frame 30 and the second frame 40 and the caught portion 62 that is provided in the other one of the first frame 30 and the second frame 40 and is caught by the latch 61. With such a configuration, the fixation of the chamber frame 3 is completed merely by hooking the latch 61 at the caught portion 62.

Moreover, in the chamber device 1 according to the present embodiment, the surface of at least one of the latch 61 and the caught portion 62 is subjected to plating treatment. With this configuration, it is possible to improve slidability and durability of the latch 61 and the caught portion 62 and to enable more smooth opening and closing operations of the chamber frame 3.

Also, in the chamber device 1 according to the present embodiment, the annular projecting portions 21A and 21B that abut the DNA array substrate 12 and the slide glass 11 in an compressed state when the chamber unit 2 is pinched by the chamber frame 3 are formed at the peripheral edge portions of the openings of the well forming portions 20. With this configuration, it is possible to form the tightly closed spaces of the well forming portions 20, that is, the reaction spaces between the DNA microarrays 12a and the reagent by the annular projecting portions 21A and 21B abutting the slide glass 11 and the DNA array substrate 12 in the compressed state.

Moreover, in the chamber device 1 according to the present embodiment, the injection ports 25 (first hole portions) and the discharge ports 24 (second hole portions) extending from the inner wall surfaces of the well forming portions 20 to the outer surfaces (the fourth surface 10D and the sixth surface 10F) are formed in the sealing member 10. With this configuration, it is possible to inject the reagent from the outer surfaces in a state where the tightly closed spaces are formed in the well forming portions 20.

Also, in the chamber device 1 according to the present embodiment, the steps 28 are formed between the injection ports 25 and the discharge ports 24 on the inner wall surfaces of the well forming portions 20. With this configuration, it is possible to suppress the reagent injected from the injection ports 25 into the well forming portions 20 being short-circuited with the discharge ports 24 immediately after the pouring and being discharged therefrom.

Moreover, the chamber device 1 according to the present embodiment includes the caps 4 (lid members) that are attached to the sealing member 10 and block the injection ports 25 and the discharge ports 24. With this configuration, it is possible to suppress the reagent injected into the well forming portions 20 leaking from the injection ports 25 or the discharge ports 24.

Also, in the chamber device 1 according to the present embodiment, the sealing member 10 includes the first engagement groove 22 that is formed on the side of the first surface 10A and is engaged with the peripheral edge portion of the slide glass 11 and the second engagement groove 29 that is formed on the side of the second surface 10B and is engaged with the peripheral edge portion of the DNA array substrate 12. With such a configuration, it is possible to retain the reagent therein without any leakage for several tens of seconds through the assembly of the whole circumferences of the slide glass 11 and the DNA array substrate 12 with the sealing member 10, and to thereby suppress the reagent scattering to the chamber frame 3 when the chamber frame 3 is discarded.

Also, the hybridization reaction system 400 according to the present embodiment performs a hybridization reaction using the chamber device 1. With this configuration, it is possible to improve operability in the hybridization reaction through an improvement in assemblability and operability of the chamber device 1.

Moreover, the nucleic acid analysis system 600 according to the present embodiment analyzes a nucleic acid extracted using the hybridization reaction system 400. With this configuration, it is possible to improve operability in the analysis of the nucleic acid through an improvement in assemblability and operability of the chamber device 1.

### [Second embodiment]

Next, a second embodiment of the present invention will be described. In the following description, configurations that are the same as or similar to those in the aforementioned embodiment will be denoted by the same reference signs, and description thereof will be simplified or omitted.

FIG. 11 is a perspective view of a well forming portion 20 according to the second embodiment of the present invention.

As illustrated in FIG. 11, a wall 28a is formed between an injection port 25 and a discharge port 24 on an inner wall surface of the well forming portion 20 according to the second embodiment.

The wall 28a extends in an oblique direction from the injection port 25 to the side of the discharge port 24. The wall 28a is formed into a tongue-like shape that is substantially triangular in a plan view. The discharge port 24 is located on the side closer to the outer surface of the sealing member 10 than a distal end of the wall 28a. With the aforementioned configuration, it is possible to suppress a reagent injected from the injection port 25 into the well forming portion 20 being short-circuited with the discharge port 24 immediately after the pouring and being discharged therefrom similarly to the step 28 in the first embodiment.

### [Third embodiment]

Next, a third embodiment of the present invention will be described. In the following description, configurations that are the same as or similar to those in the aforementioned embodiments will be denoted by the same reference signs, and description thereof will be simplified or omitted.

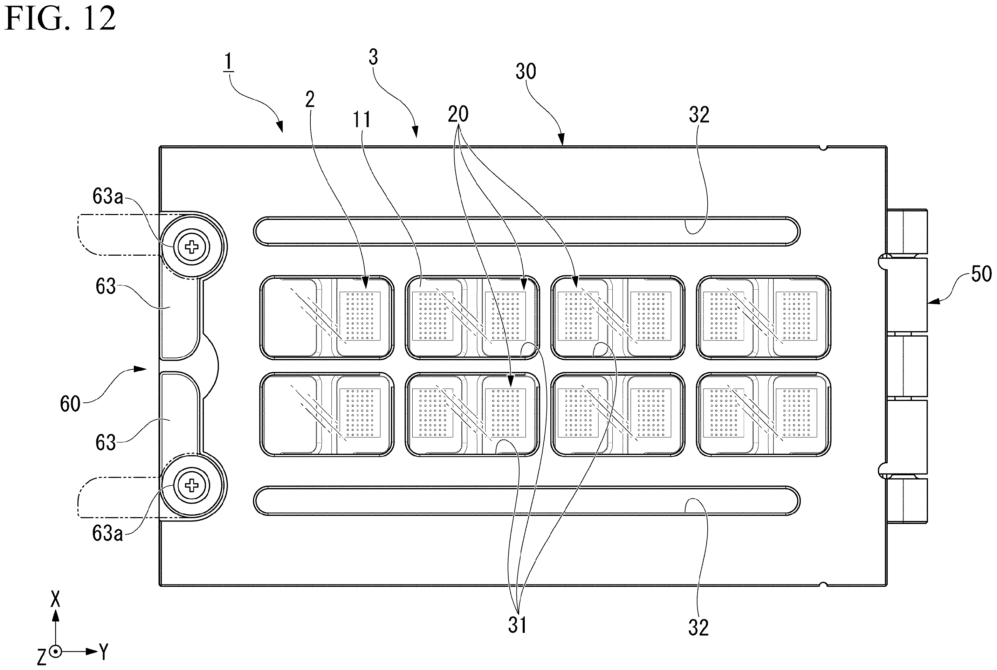

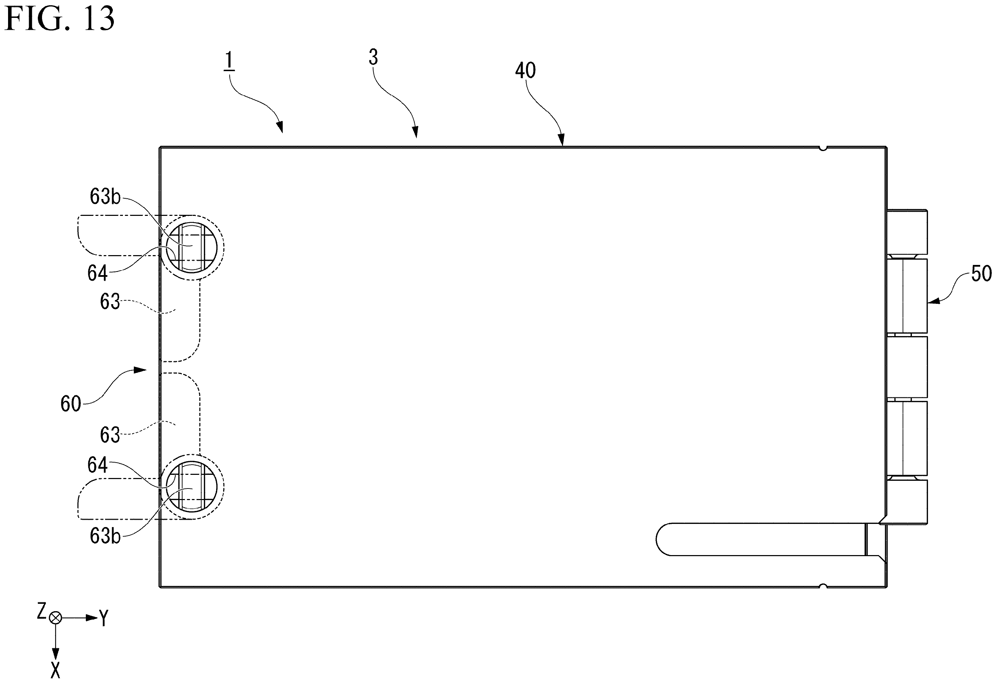

FIG. 12 is a plan view of a chamber device 1 according to a third embodiment of the present invention. FIG. 13 is a bottom view of the chamber device 1 according to the third embodiment of the present invention.

As illustrated in these drawings, a locking mechanism 60 in the third embodiment has a bayonet structure.

The locking mechanism 60 according to the third embodiment includes a pair of fixing pieces 63 that are rotatably provided in a first frame 30 and a pair of through-holes 64 that are provided in the second frame 40 and catch the pair of fixing pieces 63 through rotation. Note that the pair of fixing pieces 63 may be rotatably provided in the second frame 40 and the pair of through-holes 64 may be provided in the first frame 30.

The fixing pieces 63 are attached to a front end portion of the first frame 30 such that the fixing pieces 63 are rotatable about rotation axes 63a extending in the thickness direction (Z-axis direction) as illustrated in FIG. 12. The fixing pieces 63 include insertion ends 63b that can be inserted into the through-holes 64 as illustrated in FIG. 13. The insertion ends 63b have a substantially oblong shape in a bottom view.

The through-holes 64 penetrate through the front end portion of the second frame 40 in the thickness direction (Z-axis direction). The through-holes 64 have a substantially oblong shape extending in the front-rear direction (Y-axis direction) in a bottom view. The first frame 30 and the second frame 40 are locked by the lower ends of the fixing pieces 63 being inserted into the through-holes 64 and the insertion ends 63b being caught by opening edges of the through-holes 64 through rotation of the fixing pieces 63.

It is possible to configure the chamber device 1 by parts that are not bonded, and assemblability and operability of the chamber device 1 are improved.

### [Fourth embodiment]

Next, a fourth embodiment of the present invention will be described. **In** the following description, configurations that are the same as or similar to those in the aforementioned embodiments will be denoted by the same reference signs, and description thereof will be simplified or omitted.

In each of the aforementioned embodiments, the chamber frame 3 includes the first frame 30 that abuts the chamber unit 2 on the side of the first surface 10A, the second frame 40 that abuts the chamber unit 2 on the side of the second surface 10B, and the hinge mechanism 50 that turnably couples the first frame 30 and the second frame 40. The chamber frame 3 in each of the aforementioned embodiments is adapted on the assumption that the chamber frame 3 is repeatedly used, and the hinge mechanism 50 made of meatal is employed. On the contrary, an object of the fourth embodiment is to provide a chamber device 1 that is disposable (that can be thrown away) including the chamber frame 3.

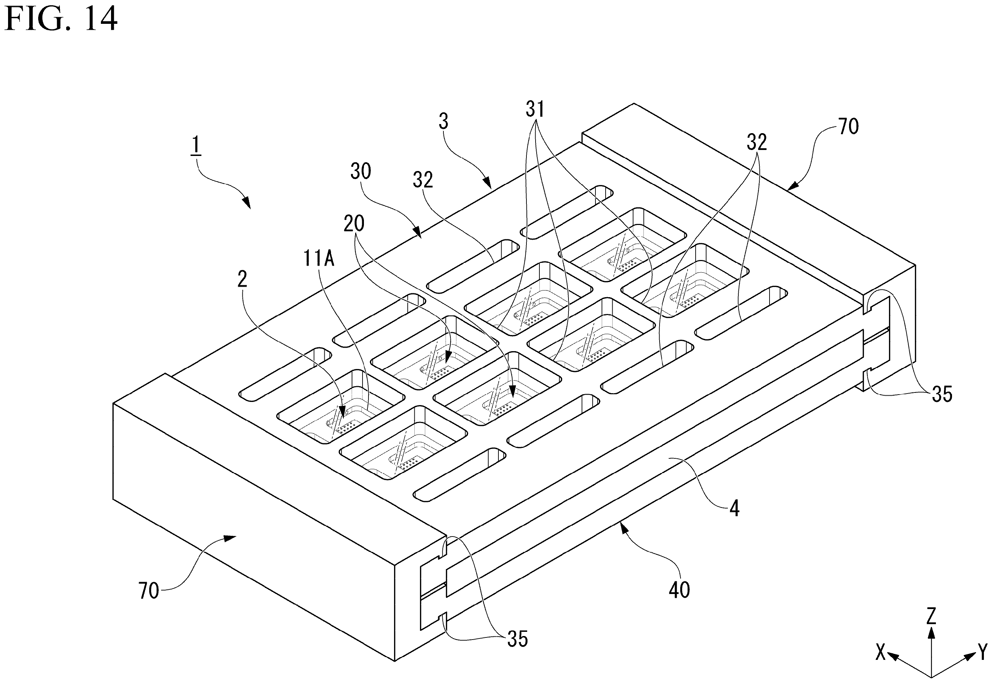

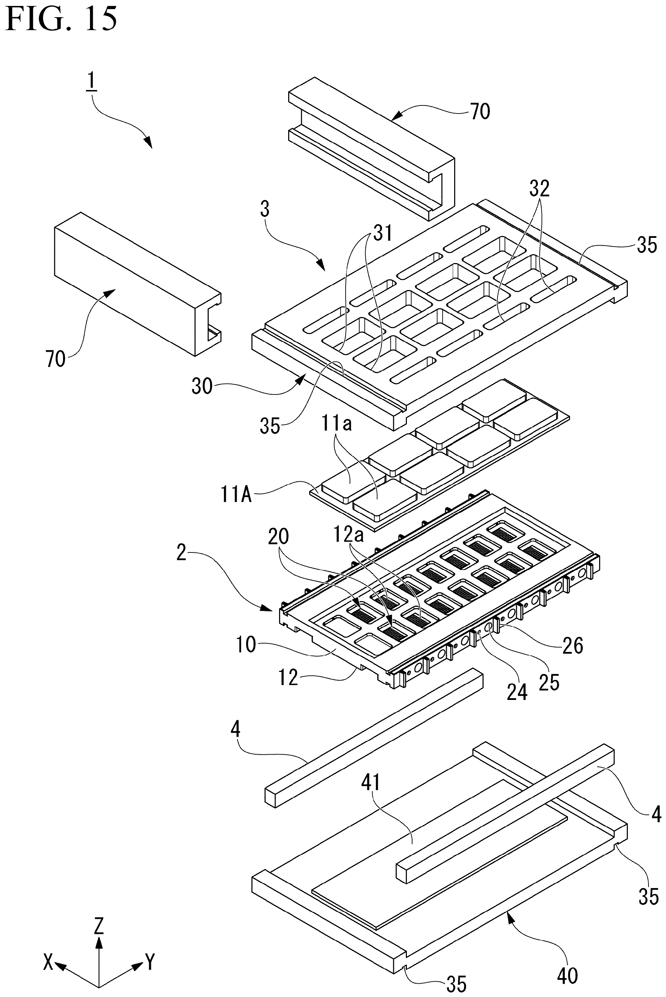

FIG. 14 is a perspective view of the chamber device 1 according to the fourth embodiment of the present invention. FIG. 15 is an exploded perspective view of the chamber device 1 according to the fourth embodiment of the present invention.

As illustrated in these drawings, the chamber device 1 according to the fourth embodiment includes clamp members 70 made of a resin and each clamping short sides of the first frame 30 and the second frame 40 on both sides in the Y-axis direction.

The clamp members 70 are formed into a U shape seen from the X-axis direction. Slide grooves 35 extending in the X-axis direction along the short sides are formed on both sides of each of the first frame 30 and the second frame 40 on both sides in the Y-axis direction. Projecting portions that are engaged with the slide grooves 35 are formed at distal end portions of the U shapes of the clamp members 70. The clamp members 70 are engaged with the first frame 30 and the second frame 40 by sliding in the X-axis direction along the slide groove 35 and pinch the first frame 30 and the second frame 40 in the Z-axis direction.

As illustrated in FIG. 15, a transparent resin member 11A is used instead of the slide glass 11 as a transparent member in the fourth embodiment. Examples of the transparent resin member 11A that can be listed include acryl, polyethylene terephthalate, polycarbonate, polyvinyl chloride. A plurality of projecting portions 11a disposed in the plurality of window portions 31 are formed in the transparent resin member 11A.

The transparent resin member 11A is integrated with the first frame 30 (high-strength resin member) through two-color molding, for example. It is possible to improve strength of the part which is hollow in each of the aforementioned embodiments by the projecting portions 11a being disposed in the window portions 31. Note that a chamber unit 2 is configured of a sealing member 10 and a DNA array substrate 12 except for the transparent member by the transparent resin member 11A being integrated with the first frame 30.

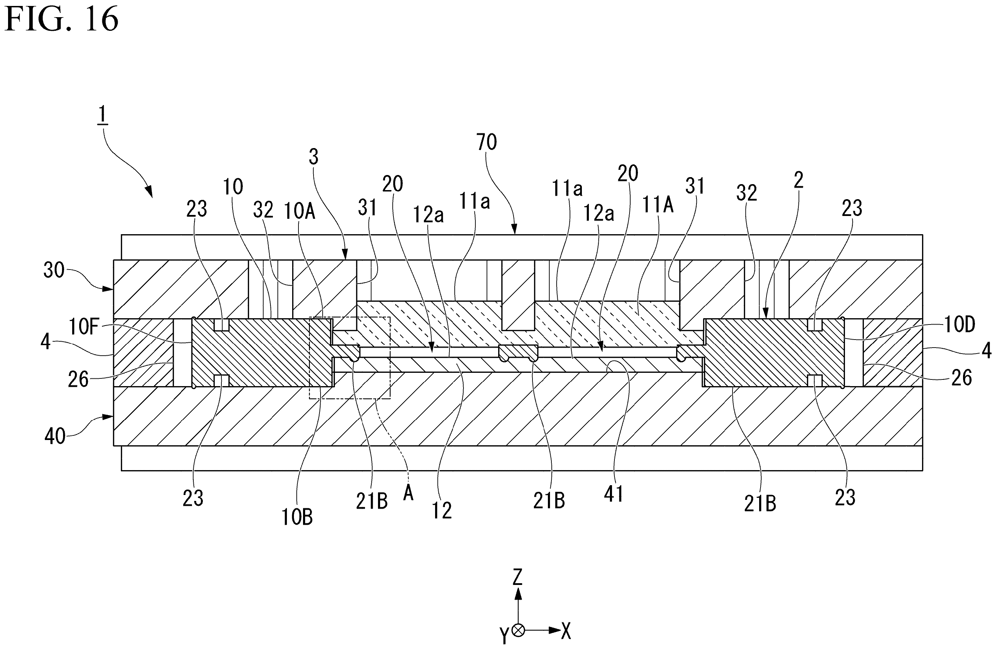

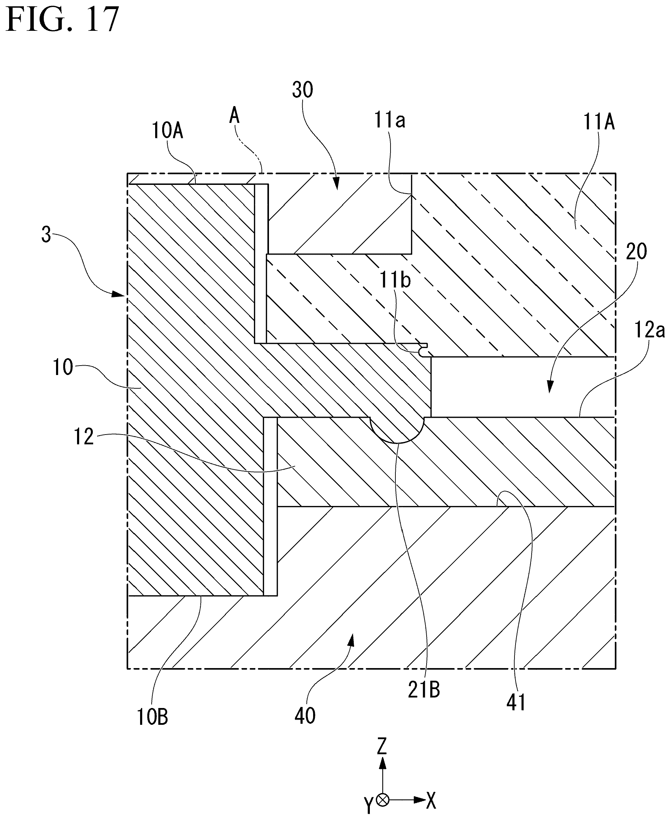

FIG. 16 is a sectional view of the chamber device 1 according to the fourth embodiment of the present invention. FIG. 17 is an enlarged view of a region A illustrated in FIG. 16.

As illustrated in FIG. 16, annular projecting portions 21B that abut the DNA array substrate 12 are formed at peripheral edge portions of openings of the well forming portions 20 on the side of the second surface 10B (-Z side) of the sealing member 10. On the other hand, the aforementioned annular projecting portions 21A in each of the aforementioned embodiments are not formed at peripheral edge portions of openings of the well forming portions 20 on the side of the first surface 10A (+Z side) of the sealing member 10.

Securing of air tightness of the well forming portions 20 on the side of the first surface 10A (+Z side) of the sealing member 10 is achieved by a flange portion 11b formed in the transparent resin member 11A as illustrated in FIG. 17. The flange portion 11b abuts the whole circumferences of inner wall surfaces of the well forming portions 20 formed by the sealing member 10. In this manner, the inner wall surfaces of the well forming portions 20 are brought into a compressed state, and it is possible to enhance air tightness of the well forming portions 20 on the side of the first surface 10A (+Z side) of the sealing member 10.

With this configuration, it is possible to reduce a force of crushing the sealing member 10 in the Z-axis direction to half the force in each of the aforementioned embodiments by providing the flange portion 11b instead of the aforementioned annular projecting portions 21A and bringing the sealing member 10 into a compressed state in the lateral direction (direction along the X-Y plane). Therefore, it is possible to sufficiently secure air tightness of the well forming portions 20 even if the hinge mechanism 50 made of metal is replaced with the clamp members 70 made of a resin. Therefore, it is possible to make the entire chamber device 1 including the chamber frame 3 disposable.

Although preferred embodiments of the present invention have been described hitherto with reference to the drawings, the present invention is not limited to the above embodiments. Various shapes, combinations, and the like of the constituent members described in the aforementioned embodiments are examples, and various modifications can be made on the basis of design requirements and the like without departing from the gist of the present invention.

Although the first frame 30 and the second frame 40 are turnably coupled by the hinge mechanism 50 in the aforementioned embodiments, for example, the hinge mechanism 50 may not be provided. In this case, the locking mechanism 60 may be provided at a part where the hinge mechanism 50 is to be placed, and the first frame 30 and the second frame 40 may be locked at the two locations.

Next, a resin chamber device, a hybridization reaction system, a nucleic acid analysis system, and a sample setting method for a resin chamber device according to fifth to seventh embodiments of the present invention will be described in detail with reference to the drawings. Hereinafter, an overview of the embodiments of the present invention will be described first, and details of each embodiment of the present invention will then be described.

### [Overview]

Japanese Unexamined Patent Application, First Publication No. 2009-082084 discloses a hybridization chamber in which flow paths for injecting test pieces are provided at two locations and the test pieces are stirred to improve reaction efficiency. The hybridization chamber is adapted not to inhibit a flow of the test pieces by an effect of a pressure alleviating section that keeps a pressure in a reaction chamber substantially constant. However, glass and a casing made of a resin are bonded in the configuration described in Japanese Unexamined Patent Application, First Publication No. 2009-082084, and manufacturing cost may thus increase. Also, it is necessary to separate glass and the casing made of a resin to discard them, and it is difficult to dispose of (throw away) them.

For the above problem, the present inventors have proposed a configuration in which a chamber unit is pinched between chamber frames coupled with the hinge mechanism such that the chamber frames can be opened and closed and the chamber frames are fixed by a latch mechanism to prevent the chamber frames from opening. With this configuration, it is possible to release the latch mechanism, to remove the chamber unit from the chamber frames, to discard the chamber unit without causing a sample to scatter by making the chamber unit disposable, and to repeatedly use the chamber frame with no need to clean it. However, the chamber frame is formed of a metal material to secure strength and the like, and the entire device has not yet been made disposable.

In the embodiments of the present invention, a transparent member, a sealing member, and a substrate are pinched between a first frame and a second frame, and peripheral edge portions of the first frame and the second frame are clamped by a clamp member. With this configuration, it is possible to secure air tightness of well forming portions formed in the sealing member while making various components using a resin. Therefore, it is possible to make the used resin chamber device disposable as it is.

### [Fifth embodiment]

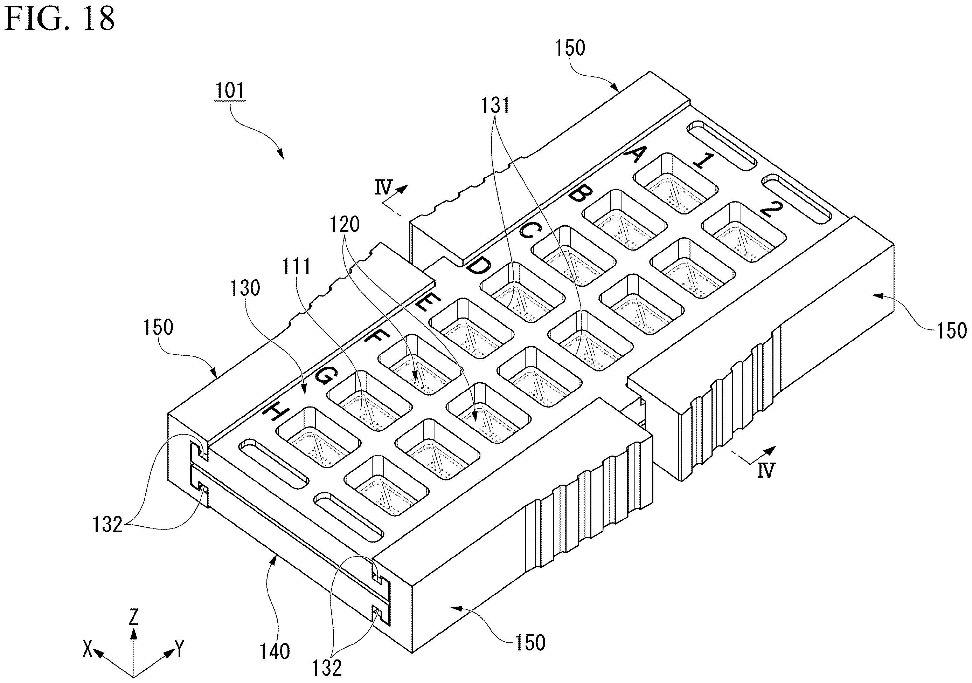

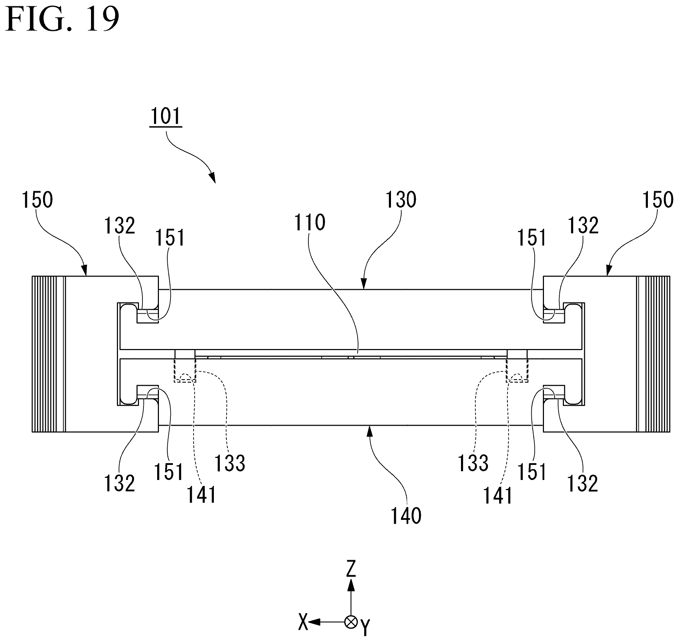

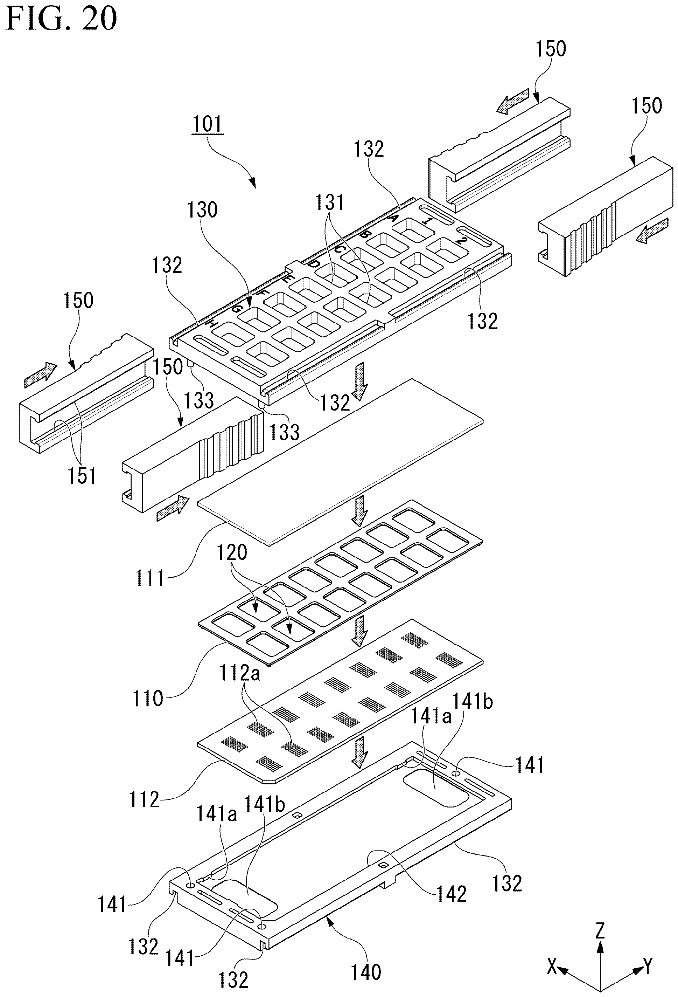

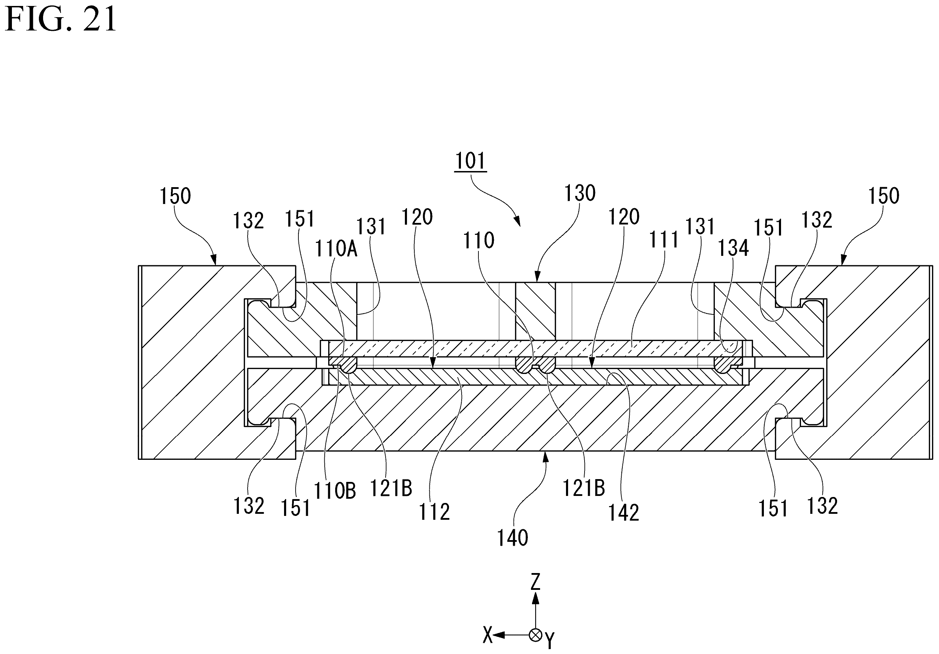

FIG. 18 is a perspective view of a chamber device 101 made of a resin according to the fifth embodiment of the present invention. FIG. 19 is a front view of the chamber device 101 made of a resin according to the fifth embodiment of the present invention. FIG. 20 is an exploded perspective view of the chamber device 101 made of a resin according to the fifth embodiment of the present invention. FIG. 21 is a sectional view of the chamber device 101 made of a resin according to the fifth embodiment of the present invention.

As illustrated in FIGS. 20 and 21, the chamber device 101 made of a resin includes a sealing member 110, a transparent member 111, a DNA array substrate 112 (substrate), a first frame 130, a second frame 140, and clamp members 150.

The sealing member 110, the transparent member 111, the DNA array substrate 112, the first frame 130, the second frame 140, and the clamp members 150 are formed of a resin material. The sealing member 110 may be formed of a silicone resin with elasticity, for example, to secure air tightness of the well forming portions 120. The first frame 130, the second frame 140, and the clamp members 150 are formed of a resin material with higher strength than that of the sealing member 110 to keep the shapes. As the resin material with high strength, a resin material with an elastic modulus of equal to or greater than 10 GPa is preferably used, and it is possible to use known resin materials unless otherwise particularly explained. Note that although the transparent member 111 and the DNA array substrate 112 may be formed of a resin material with high strength, they may be formed of a resin material with an elastic modulus of about 2 GPa (for example, a cycloolefin polymer (COP) resin).

The chamber device 101 made of a resin has an oblong shape in a plan view as illustrated in FIG. 18. Note that an XYZ orthogonal coordinate system may be set, and positional relationships of members may be explained with reference to the XYZ orthogonal coordinate system in the following description. The X-axis direction is a short-side direction (left-right direction) of the chamber device 101 made of a resin. The Y-axis direction is a long-side direction (front-rear direction) of the chamber device 101 made of a resin. The Z-axis direction is a thickness direction (up-down direction) of the chamber device 101 made of a resin.

As illustrated in FIG. 20, the sealing member 110 has a flat shape that is rectangular in a plan view. A plurality of well forming portions 120 penetrating through the sealing member 110 in the thickness direction (Z-axis direction) are formed in the sealing member 110. The well forming portions 120 form columns in the long-side direction (Y-axis direction) and are formed in two columns at an interval in the short-side direction (X-axis direction). Note that the number, the arrangement, and the shape of the well forming portions 120 are not limited to those illustrated in the drawing.

The transparent member 111 is disposed on the sealing member 110 on the side of a first surface 110A from which ends (upper ends) of the well forming portions 120 on one side are open as illustrated in FIG. 21. The transparent member 111 blocks the openings at the ends (upper ends) of the well forming portions 120 on the one side in a state where the chamber device 101 made of a resin is assembled. As the transparent member 111, it is possible to suitably use a cycloolefin polymer (COP) resin, for example. Note that the sealing member 110 may be molded in advance on one surface of the transparent member 111. In this manner, it is possible to enhance sealability between the transparent member 111 and the sealing member 110.

The DNA array substrate 112 includes a plurality of DNA microarrays 112a in which probe carriers to cause a hybridization reaction are arrange in an array shape as illustrated in FIG. 20. The DNA microarrays 112a are formed in an arrangement corresponding to the well forming portions 120. Note that the number of formed DNA microarrays 112a may not necessarily be the same as the number of well forming portions 120. Also, in a case where a reaction other than the hybridization reaction is performed, a substrate including reaction portions corresponding thereto may be used.

The DNA array substrate 112 is disposed on the sealing member 110 on the side of a second surface 110B from which ends (lower ends) of the well forming portions 120 on the other side are open as illustrated in FIG. 21. The DNA array substrate 112 blocks the ends (lower ends) of the well forming portions 120 on the other side in a state where the chamber device 101 made of a resin is assembled. Annular projecting portions 121B that abut the DNA array substrate 112 are formed at peripheral edge portions of the openings of the well forming portions 120 on the side of the second surface 110B of the sealing member 110. The annular projecting portions 121B have a projecting shape that is semicircular in a sectional view. The annular projecting portions 121B enhance air tightness of the well forming portions 120 by abutting the DNA array substrate 112 attached to the sealing member 110 in a compressed state in the up-down direction (Z-axis direction).

A plurality of window portions 131 for observing the well forming portions 120 are formed in the first frame 130 as illustrated in FIG. 20. The plurality of window portions 131 are formed at positions where the window portions overlap the well forming portions 120 in the Z-axis direction in a state where the chamber device 101 made of a resin is assembled. The window portions 131 penetrate through the first frame 130 in the thickness direction (Z-axis direction). The window portions 131 form columns in the long-side direction (Y-axis direction) and are formed in two columns at an interval in the short-side direction (X-axis direction). Note that the number, arrangement, and the shape of the window portions 131 are not limited to those illustrated in the drawing. A groove portion 134 where the transparent member 111 that blocks the window portions 131 are disposed is formed on the lower side (-Z side) of the first frame 130 as illustrated in FIG. 21.

The surface of the first frame 130 is covered with jet black plating made of a resin. In a case where the first frame 130 is formed of an ordinary high-strength resin material, the first frame 130 may cause autofluorescence and inhibit fluorescence observation in the well forming portions 120. Therefore, it is possible to prevent the fluorescence observation from being inhibited due to autofluorescence of the first frame 130 by covering the surface of the first frame 130 with the jet black plating made of a resin. Note that the sealing member 110, the transparent member 111, and the DNA array substrate 112 forming the well forming portions 120 in addition to the first frame 130 may also be formed of a resin material of low autofluorescence of equal to or less than 5 uW/m², for example.

Positioning pins 133 are formed in the first frame 130 on the side (-Z side) of the surface facing the second frame 140 as illustrated in FIGS. 19 and 20. Positioning holes 141 with which the positioning pins 133 are engaged are formed in the second frame 140 on the side (+Z side) of the surface facing the first frame 130. The positioning pins 133 and the positioning holes 141 are provided at two locations on one side (-Y side) in the long-side direction and one location on the other side (+Y side) in the long-side direction as illustrated in FIG. 20, and the positioning pins 133 and the positioning holes 141 cannot be assembled in a case where the first frame 130 and the second frame 140 are in the opposite orientations in the long-side direction.

An accommodating groove 142 that accommodates the DNA array substrate 112 is formed in the second frame 140 as illustrated in FIG. 20. The accommodating groove 142 has a rectangular shape in a plan view. A plurality of projecting portions 141a that position the DNA array substrate 112 are formed in side surfaces of the accommodating groove 142. Also, recessed portions 141b that are slightly lowered are formed in a bottom surface of the accommodating groove 142. Double-sided adhesive tapes, which are not illustrated, to fix the DNA array substrate 112 are disposed in the recessed portions 141b.

The first frame 130 and the second frame 140 are formed into an oblong shape in a plan view as illustrated in FIG. 20. Slide grooves 132 extending in the long-side direction (Y-axis direction) are formed at peripheral edge portions of the first frame 130 and the second frame 140. The slide grooves 132 are formed along a pair of long sides of each of the first frame 130 and the second frame 140 from four corner portions of each of the first frame 130 and the second frame 140. The slide grooves 132 are linearly formed at a constant depth in the upper surface of the first frame 130 and the lower surface of the second frame 140.

Clamp members 150 are engaged with the slide grooves 132 as illustrated in FIGS. 18 and 19. The clamp members 150 are engaged with the slide grooves 132 from the four corner portions of each of the first frame 130 and the second frame 140. Therefore, the peripheral edge portions of the first frame 130 and the second frame 140 are clamped by the four clamp members 150 in the present embodiment.

The clamp members 150 are formed into a U shape seen from the long-side direction (Y-axis direction) as illustrated in FIG. 19. Engagement claw portions 151 that are engaged with the slide grooves 132 are formed at distal end portions of the U shapes of the clamp members 150. The clamp members 150 are engaged with the first frame 130 and the second frame 140 by sliding in the Y-axis direction along the slide grooves 132 and clamp the first frame 130 and the second frame 140 in the thickness direction.

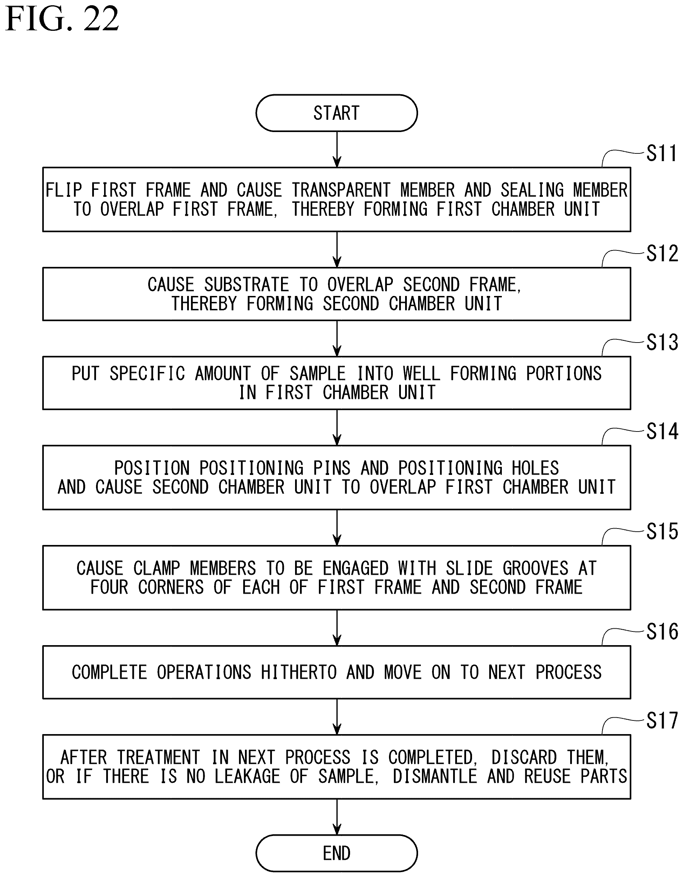

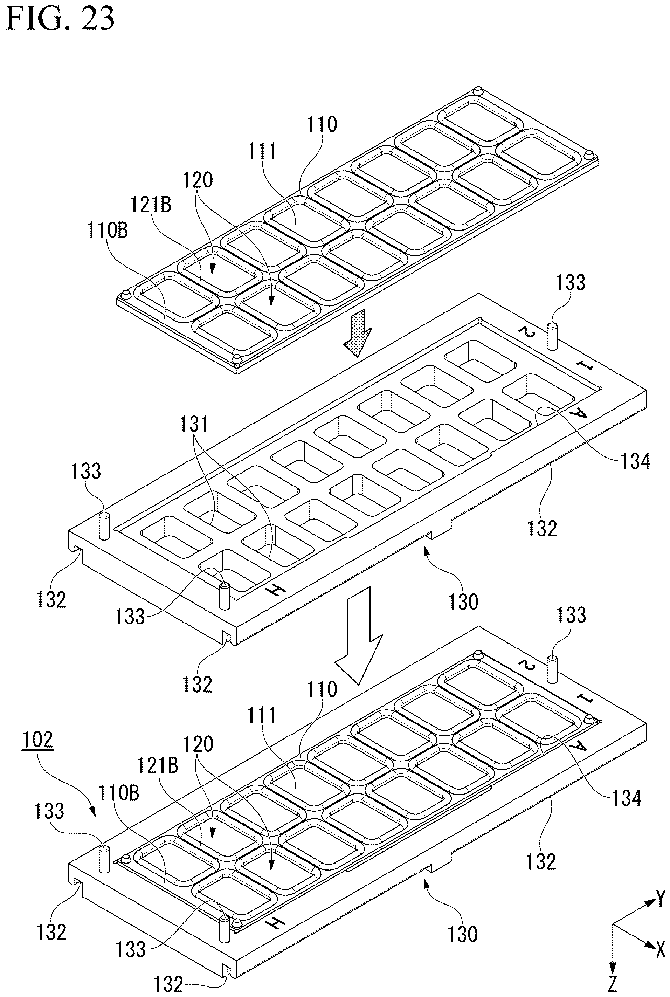

FIG. 22 is a flow diagram of a sample setting method for the chamber device 101 made of a resin according to the fifth embodiment of the present invention. FIGS. 23 to 26 are assembly diagrams of the chamber device 101 made of a resin in accordance with the flow diagram illustrated in FIG. 22.

In a case where a sample is set in the aforementioned chamber device 101 made of a resin, a first chamber unit 102 is formed as illustrated in FIG. 23 first (first process). Specifically, the first frame 130 is flipped, and the transparent member 111 and the sealing member 110 are caused to overlap the first frame 130, thereby forming a first chamber unit 102 (Step S1).

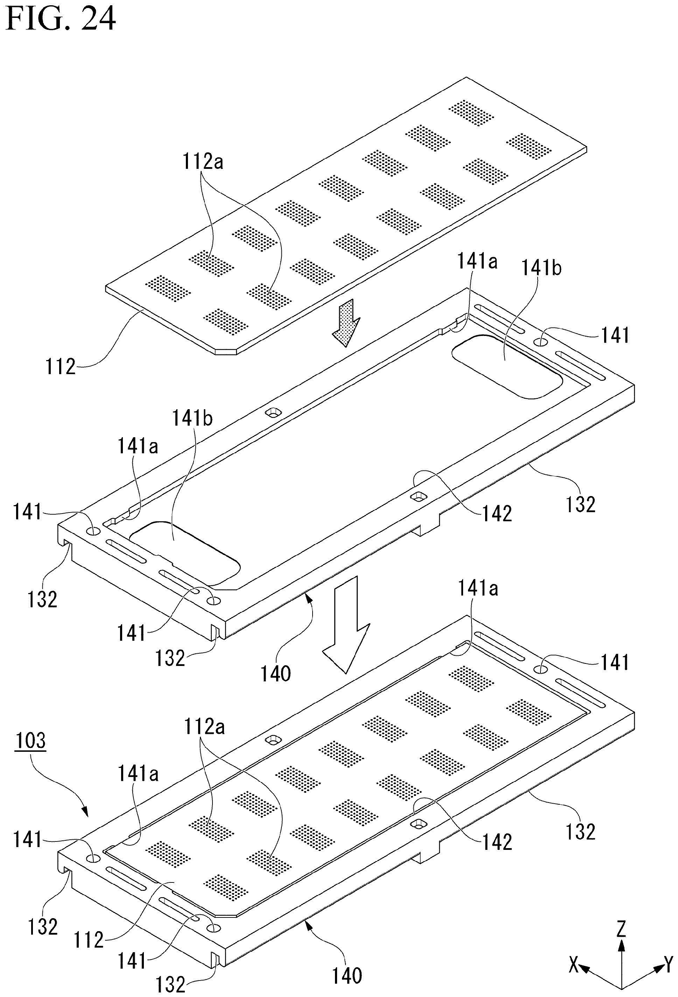

Next, a second chamber unit 103 is formed as illustrated in FIG. 24. Specifically, the DNA array substrate 112 is caused to overlap the second frame 140, thereby forming a second chamber unit 103 (Step S2). Note that double-sided adhesive tapes, which are not illustrated, may be disposed in the accommodation groove 142 of the second frame 140, and the DNA array substrate 112 may be fixed thereto.

Next, specific amounts of samples are placed into the well forming portions 120 in the first chamber unit 102 illustrated in FIG. 23 and assembled in Step S1 (Step S3). Specifically, the samples are dropped from the side (-Z side) of the ends of the openings of the well forming portions 120 on the other side into the well forming portions 120 using a pipettor or the like (second process). It is possible to drop the samples immediately above the well forming portions 120, and an operators can thus perform the operation with no need of any special skills. Also, since the annular projecting portions 121B are formed at the opening peripheral edge portions of the well forming portions 120, the probability that contamination occurs between adjacent well forming portions 120 is considerably low.

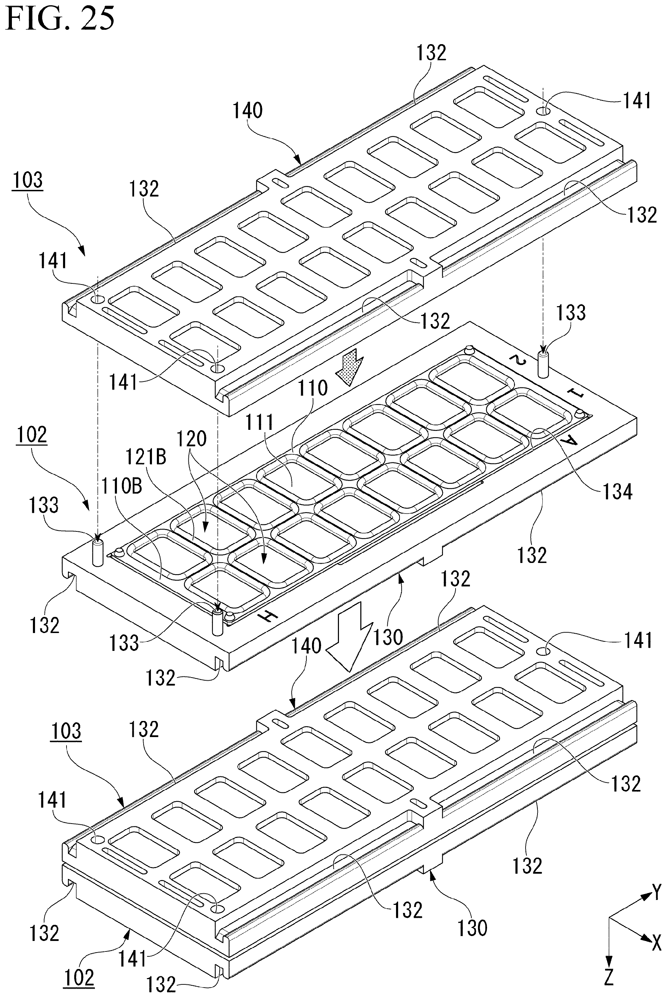

Next, the second chamber unit 103 is flipped and is caused to overlap the first chamber unit 102 as illustrated in FIG. 25 (Step S4). Specifically, the positioning pins 133 of the first frame 130 and the positioning holes 141 of the second frame 140 are positioned, and the second chamber unit is thereby caused to overlap the first chamber unit. Although the second chamber unit 103 is flipped at this time, the DNA array substrate 112 is fixed to the second frame 140, and the DNA array substrate 112 does not drop. In this manner, the ends of the well forming portions 120 on the other side are blocked by the DNA array substrate 112 by causing the second chamber unit 103 to overlap the first chamber unit 102 (third process).

Since the combination of the DNA array substrate 112 is performed after dropping the samples in this manner, contact between the DNA array substrate 112 and the pipettor does not occur, and there is no concern of damaging the DNA microarrays 112a on the DNA array substrate 112 at all. Also, it is possible to perform the assembly with high precision by fitting the positioning pins 133 of the first frame 130 to the positioning holes 141 of the second frame 140 to combine the DNA array substrate 112.

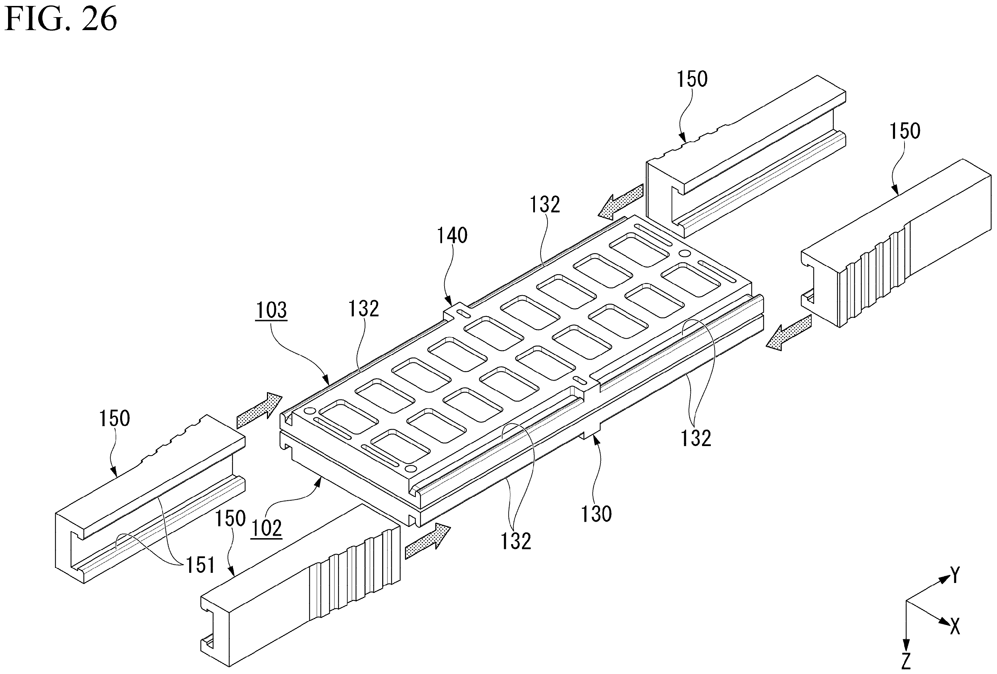

Next, the peripheral edge portions of the first frame 130 and the second frame 140 are clamped by the clamp members 150 as illustrated in FIG. 26 (fourth process). Specifically, the clamp members 150 are engaged with the slide grooves 132 at the four corners of the first frame 130 and the second frame 140 (Step S5). In this manner, the sealing member 110 is compressed between the first frame 130 and the second frame 140, and the well forming portions 120 are brought into a tightly closed state as illustrated in FIG. 21. Once all the aforementioned processes are completed, the processing moves on to the next process (Step S6).

After treatment in the next process is completed, the chamber device 101 made of a resin that has finished its role can be discarded as it is (Step S7). The chamber device 101 made of a resin does not contain any metal material or the like and can thus be discarded without separation. Note that the components of the chamber device 101 made of a resin may be dismantled and some of the components may be reused as long as there is no leakage of the samples.

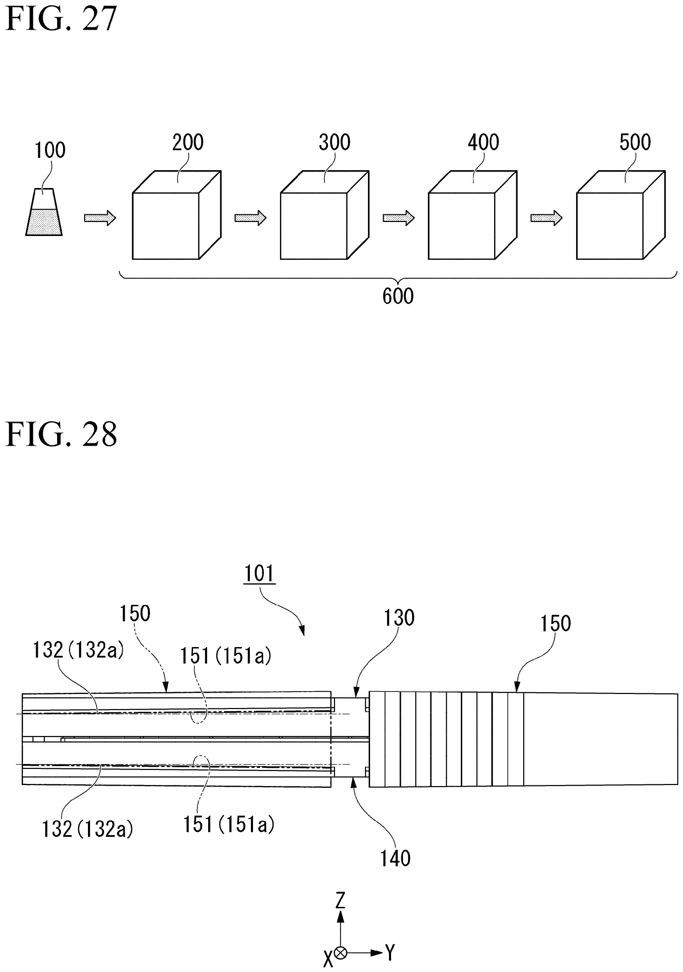

FIG. 27 is a schematic view of the nucleic acid analysis system 600 using the chamber device 101 made of a resin according to the fifth embodiment of the present invention.

As illustrated in FIG. 27, the nucleic acid analysis system 600 includes a germ recovery system 200, a nucleic acid extraction system 300, a hybridization reaction system 400, and a detection system 500.

The germ recovery system 200 is a system that collects germs (such as bacteria and fungi) contained in a sample 100 from the sample 100. The sample 100 is, for example, a manufactured beverage, water to manufacture the beverage, or a solution in a process of manufacturing the beverage in a case of an inspection targeted at a beverage. Alternatively, the sample 100 may be a solution obtained by collecting germs from a cotton swab wiping off an inspection environment in order to inspect whether or not a manufacturing environment is contaminated by germs or a degree of contamination.

The germs can be collected through filtration by a filter or the like by pressurizing or depressurizing the collected solution, for example. In a case where bacteria or fungi are collected, for example, the pore diameter of the filter may be 0.22 µm to 0.45 µm. After germs are collected by the filter, the filter is accommodated in a culture container, which will be described later, and is dipped into a culture solution in which the germs are cultivated to thereby cultivate the germs. The cultivation of the germs is, for example, static culture in which the culture container is stationarily placed to cultivate the germs or shaking culture in which the cultivation is performed while the culture container is shaken. The culture solution with the germs cultivated therein is transferred to the next process (nucleic acid extraction system 300). Note that the germs may be collected through centrifugation and be transferred to the next process, or the solution with the filter therein may be shaken and the solution with germs suspended therein may be transferred to the next process.

The nucleic acid extraction system 300 is a system that breaks (dissolves) membrane structures in cells in the solution and extracts nucleic acids of the cells of the germs. Note that a solution containing a different nucleic acid that reacts with the extracted nucleic acid may be mixed into the sample 100 from which the nucleic acid has been extracted. Also, the different nucleic acid may be a nucleic acid to which a site that exhibits a fluorescent, light-emitting, or light extinction effect under a specific condition is applied in order to be detected in a detection process (detection system 500), which will be described later. These may be mixed into the sample 100 before the nucleic acid extraction system 300 performs the treatment, or may be mixed into the sample 100 after the nucleic acid extraction system 300 performs the treatment.

The hybridization reaction system 400 is a system that causes the nucleic acid in the sample 100 to cause a hybridization reaction. In the process, the aforementioned chamber device 101 made of a resin is used. In the hybridization reaction, the sample 100 causes a hybridization reaction that matches the aforementioned different nucleic acid by being heated to 60°C, for example, and stirred in the well forming portions 120. Through the reaction, the site that exhibits the fluorescent, light emitting, or light extinction effect under the specific condition applied to the aforementioned different nucleic acid reacts the nucleic acid in the sample 100, for example, and the fluorescent, light-emitting, or light extinction effect is thereby expressed.

Note that it is possible to cause the aforementioned different nucleic acid to react only with a nucleic acid that a specific germ in the sample 100 has, for example, by designing the structure of the aforementioned different nucleic acid to react with the specific nucleic acid. In other words, it is possible to express the fluorescent, light-emitting, or light extinction effect applied to the different nucleic acid only when the sample 100 contains the specific germ by using the different nucleic acid that reacts with the specific nucleic acid in the treatment performed by the hybridization reaction system 400.

The detection system 500 detects whether or not the fluorescent, light-emitting, or light extinction effect has been expressed in the sample 100 treated by the hybridization reaction system 400, a degree thereof, and the like. The detection system 500 excites the fluorescent effect expressed by the nucleic acid in the sample 100 with excitation laser light, for example, and the fluorescent light after the excitation is detected by a high-sensitivity camera.

Alternatively, the detection system 500 detects the light-emitting effect expressed by the nucleic acid in the sample 100 by a high-sensitivity camera. Alternatively, the detection system 500 detects a degree of light extinction of the fluorescent light or emitted light applied to the vicinity of the site to which the light extinction effect is applied by a high-sensitivity camera. In regard to the detection method, a method as described in Japanese Unexamined Patent Application, First Publication No. 2020-74726, for example, may be employed.

The nucleic acid analysis system 600 can analyze whether a specific germ (such as bacteria or fungi) is contained in the sample 100 or the concentration thereof by using the series of systems as described above.

As described above, the chamber device 101 made of a resin according to the present embodiment includes: the sealing member 1 10 in which the well forming portions 120 penetrating through the sealing member 110 in the thickness direction are formed; the transparent member 111 that is disposed on the sealing member 110 on the side of the first surface 110A from which ends of the well forming portions 120 on the one side are open, the transparent member 111 blocking the ends of the well forming portions 120 on the one side; the DNA array substrate 112 that is disposed on the sealing member on the side of the second surface 110B from which ends of the well forming portions 120 on the other side, the DNA array substrate 112 blocking the ends of the well forming portions 120 on the other side; the first frame 130 that abuts the transparent member 111; the second frame 140 that abuts the DNA array substrate 112; and the clamp members 150 that clamp the peripheral edge portions of the first frame 130 and the second frame 140. With this configuration, it is possible to secure air tightness of the well forming portions 120 and to make the entire chamber device 101 made of a resin disposable.

Also, in the chamber device 101 made of a resin according to the present embodiment, the first frame 130 and the second frame 140 are formed into oblong shapes in a plan view, and the plurality of slide grooves 132 extending in the long-side direction from four corner portions and allowing the clamp members 150 to be engaged therewith are formed therein. With this configuration, it is possible to secure air tightness of the well forming portions 120 by clamping the four corners of each of the first frame 130 and the second frame 140 without using any metal materials. Note that as for air tightness of the well forming portions 120 achieved by the chamber device 101 made of a resin, it has been confirmed to be possible to secure air tightness that is equivalent to that of the configuration using the chamber frame made of the metal material explained in [Overview] in leakage tests.

Also, in the chamber device 101 made of a resin according to the present embodiment, the surface of the first frame 130 is covered with jet black plating made of a resin, and the window portions 131 for looking into the well forming portions 120 through the transparent member 111 are formed therein. With this configuration, it is possible to prevent fluorescence observation from being inhibited due to autofluorescence of the first frame 130.

Also, in the chamber device 101 made of a resin according to the present embodiment, one of the first frame 130 and the second frame 140 includes the positioning pins 133 projecting toward the other one formed therein, and the positioning holes 141 with which the positioning pins 133 are engaged are formed in the other one of the first frame 130 and the second frame 140. With this configuration, it is possible to combine the first frame 130 and the second frame 140 with high precision.

Also, the hybridization reaction system 400 according to the present embodiment performs a hybridization reaction using the resin chamber device. With this configuration, it is possible to make the entire chamber device 101 made of a resin disposable and to thereby improve operation efficiency of the hybridization reaction.

Moreover, the nucleic acid analysis system 600 according to the present embodiment analyzes a nucleic acid extracted using the hybridization reaction system 400. With this configuration, it is possible to make the entire chamber device 101 made of a resin disposable and to thereby improve operation efficiency of the analysis of the nucleic acid.

Also, the sample setting method for the chamber device 101 made of a resin according to the present embodiment includes: a first process of forming the first chamber unit by causing the sealing member 110 in which ends of well forming portions 120 on one side are blocked with the transparent member 111 to overlap the first frame 130, the well forming portions 120 penetrating through the sealing member 110 in the thickness direction; a second process of dropping a sample from ends of the well forming portions 120 on the other side into the well forming portions 120 after the first process; a third process of causing the second chamber unit formed by causing the DNA array substrate 112 to overlap the second frame 140 to overlap the first chamber unit and blocking the ends of the well forming portions 120 on the other side with the DNA array substrate 112 after the second process; and a fourth process of clamping the peripheral edge portions of the first frame 130 and the second frame 140 with the clamp members 150 after the third process. With this configuration, the DNA array substrate 12 is combined after the sample is dropped, the combination of the DNA array substrate 112 is performed after dropping the samples, contact between the DNA array substrate 112 and the pipettor does not occur, and there is no concern of damaging the DNA microarrays 112a on the DNA array substrate 112 at all. Also, it is possible to drop the sample from immediately above the well forming portions 120, and an operator can perform the operation with no need of special skills.

### [Sixth embodiment]

Next, a sixth embodiment of the present invention will be described. In the following description, configurations that are the same as or similar to those in the aforementioned embodiments will be denoted by the same reference signs, and description thereof will be simplified or omitted.

FIG. 28 is a side view of a chamber device 101 made of a resin according to the sixth embodiment of the present invention.

As illustrated in FIG. 28, slide grooves 132 in the sixth embodiment are inclined. Specifically, the slide grooves 132 include a first inclined shape 132a with a depth in the thickness direction (Z-axis direction) decreasing toward an intermediate position of a first frame 130 and a second frame 140 in the long-side direction (Y-axis direction).

The first inclined shape 132a is formed with an inclination of about 0.5° with respect to the X-Y plane. In other words, the inlet dimension of the upper and lower slide grooves 132 in the Z-axis direction is small, and the dimension thereof in the Z-axis direction increases toward the further side in the state where the first frame 130 and the second frame 140 are combined. Clamp members 150 include engagement claw portions 151 that have a second inclined shape 151a corresponding to the first inclined shape 132a. In other words, the second inclined shape 151a is also formed with an inclination of about 0.5° with respect to the X-Y plane.

With this configuration, the slide grooves 132 and the engagement claw portions 151 have the inclined shapes in the same orientation, and it is possible to absorb variations in dimensions of the resin material parts. In other words, since the inlet dimension of the slide grooves 132 in the state where the first frame 130 and the second frame 140 are combined is small, and the inlet dimension of the clamp members 150 is large on the contrary, the clamp members 150 can be smoothly engaged with the slide grooves 132. Also, once the clamp member 150 is inserted into the slide groove 132 on the further side, both the first inclined shape 132a and the second inclined shape 151a are brought into a close contact with each other, and gaps are thus unlikely to be generated between the clamp members 150 and the slide grooves 132 even if there are variations in dimensions of the resin material parts.

### [Seventh embodiment]

Next, a seventh embodiment of the present invention will be described. In the following description, configurations that are the same as or similar to those in the aforementioned embodiments will be denoted by the same reference signs, and description thereof will be simplified or omitted.

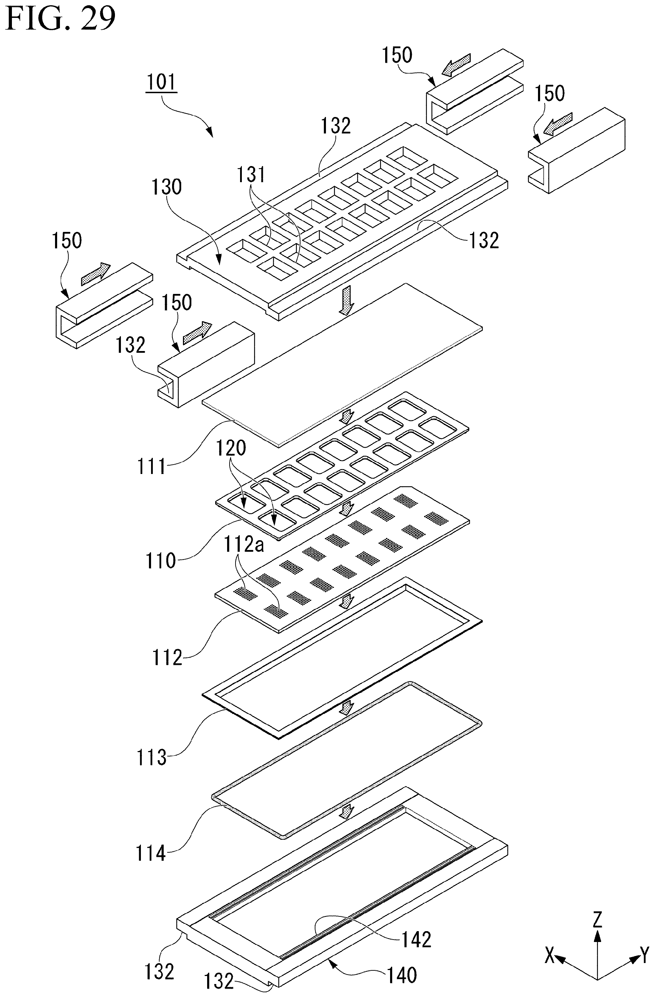

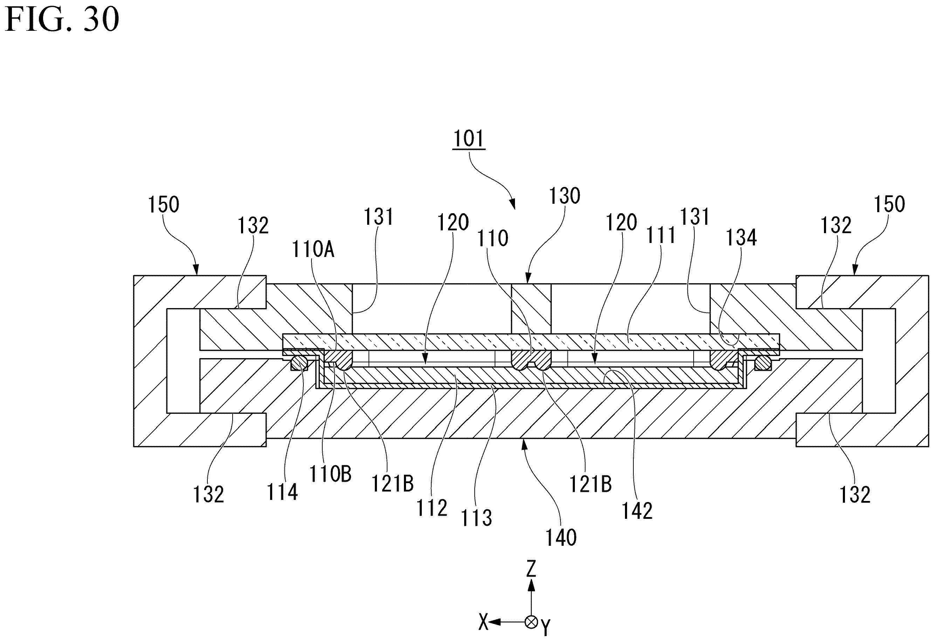

FIG. 29 is an exploded perspective view of a chamber device 101 made of a resin according to the seventh embodiment of the present invention. FIG. 30 is a sectional view of the chamber device 101 made of a resin according to the seventh embodiment of the present invention.

As illustrated in these drawings, the chamber device 101 made of a resin according to the seventh embodiment includes a sample receiving member 113 and a gasket 114 in addition to the sealing member 110, the transparent member 111, the DNA array substrate 112, the first frame 130, the second frame 140, and the clamp members 150 described above. Note that the sample receiving member 113 and the gasket 114 are also formed of a resin material.

The sample receiving member 113 has a dish shape that accommodates the DNA array substrate 112 as illustrated in FIG. 30. The entire edge portion of the dish shape of the sample receiving member 113 is caused to adhere to the outer peripheral edge of the transparent member 111 via an adhesive tape of an adhesive agent, which is not illustrated. The gasket 114 is formed into a rectangular annular shape and is disposed on the -Z side of the edge portion of the dish shape of the sample receiving member 113. The gasket 114 is brought into a compressed state between the first frame 130 and the second frame 140 and pushes the edge portion (adhesive portion) of the dish shape of the sample receiving member 113 against the transparent member 111 with a reaction force thereof.

With this configuration, it is possible to receive a sample spilled from the well forming portions 120 by the sample receiving member 113 when the chamber device 101 made of a resin is dismantled. It is thus possible to reuse the first frame 130, the second frame 140, and the clamp members 150 without cleaning them.

Although the preferred embodiments of the present invention have been described hitherto with reference to the drawings, the present invention is not limited to the aforementioned embodiments. Various shapes, combinations, and the like of the constituent members described in the aforementioned embodiments are examples, and various modifications can be made on the basis of design requirements and the like without departing from the gist of the present invention.

## Claims

1. A resin chamber device comprising:
a sealing member in which well forming portions penetrating through the sealing member in a thickness direction are formed;
a transparent member that is disposed on the sealing member on a side of a first surface from which ends of the well forming portions on one side are open, the transparent member blocking the ends of the well forming portions on the one side;
a substrate that is disposed on the sealing member on a side of a second surface from which ends of the well forming portions on the other side are open, the substrate blocking the ends of the well forming portions on the other side;
a first frame that abuts the transparent member;
a second frame that abuts the substrate; and
a clamp member that clamps peripheral edge portions of the first frame and the second frame.

2. The resin chamber device according to claim 1,
wherein the first frame and the second frame are formed into rectangular shapes in a plan view, and a plurality of slide grooves that extend in a long-side direction from four corner portions and allow the clamp member to be engaged with the slide grooves are formed.

3. The resin chamber device according to claim 2,
wherein the slide grooves have a first inclined shape that becomes shallower toward an intermediate position of the first frame and the second frame in the long-side direction, and
wherein the clamp member has an engagement claw portion that is engaged with the slide grooves and has a second inclined shape corresponding to the first inclined shape.

4. The resin chamber device according to any one of claims 1 to 3,
wherein a surface of the first frame is covered with jet black plating made of a resin, and a window portion to look into the well forming portions through the transparent member is formed in the first frame.

5. The resin chamber device according to any one of claims 1 to 3,
wherein one of the first frame and the second frame includes a positioning pin formed thereon to project toward the other, and
wherein a positioning hole with which the positioning pin is engaged is formed in the other one of the first frame and the second frame.

6. A hybridization reaction system that performs a hybridization reaction using the resin chamber device according to any one of claims 1 to 3.

7. A nucleic acid analysis system that analyzes a nucleic acid extracted using the hybridization reaction system according to claim 6.

8. A sample setting method for a resin chamber device, comprising:
a first process of forming a first chamber unit by causing a sealing member in which ends of well forming portions on one side are blocked with a transparent member to overlap a first frame, the well forming portions penetrating through the sealing member in a thickness direction;
a second process of dropping a sample from ends of the well forming portions on the other side into the well forming portions after the first process;
a third process of causing a second chamber unit formed by causing a substrate to overlap a second frame to overlap the first chamber unit and blocking the ends of the well forming portions on the other side with the substrate after the second process; and
a fourth process of clamping peripheral edge portions of the first frame and the second frame with a clamp member after the third process.
